(19) Europäisches Patentamt
European Patent Office
Office européen des brevets

(11) EP 1 910 335 B1

(12) **FASCICULE DE BREVET EUROPEEN**

(45) Date de publication et mention de la délivrance du brevet:
**06.11.2013 Bulletin 2013/45**

(21) Numéro de dépôt: **06778898.4**

(22) Date de dépôt: **20.07.2006**

(51) Int Cl.:
***C07D 401/14*** *(2006.01)* ***C07D 403/14*** *(2006.01)*
***C07D 413/14*** *(2006.01)* ***C07D 417/14*** *(2006.01)*
***A61K 31/4178*** *(2006.01)* ***A61K 31/4184*** *(2006.01)*
***A61K 31/427*** *(2006.01)* ***A61K 31/4439*** *(2006.01)*
***A61K 31/444*** *(2006.01)* ***A61P 17/00*** *(2006.01)*
***A61K 31/4709*** *(2006.01)* ***A61K 31/5355*** *(2006.01)*

(86) Numéro de dépôt international:
**PCT/FR2006/001782**

(87) Numéro de publication internationale:
**WO 2007/010144 (25.01.2007 Gazette 2007/04)**

(54) **DÉRIVÉS DE N-(HÉTÉROARYL)-1-HÉTÉROARYLALKYL-1H-INDOLE-2-CARBOXAMIDES, LEUR PRÉPARATION ET LEUR APPLICATION EN THÉRAPEUTIQUE**

N-(HETEROARYL)-1-HETEROARYLAKYL-1H-INDOL-2-CARBONSÄUREAMIDDERIVATE, DEREN HERSTELLUNG UND VERWENDUNG

N-(HETEROARYL)-1-HETEROARYLALKYL-1H-INDOLE-2-CARBOXAMIDE DERIVATIVES, PREPARATION AND USE THEREOF

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **22.07.2005 FR 0507803**

(43) Date de publication de la demande:
**16.04.2008 Bulletin 2008/16**

(73) Titulaire: **SANOFI**
**75008 Paris (FR)**

(72) Inventeurs:
- **DUBOIS, Laurent**
**F-92350 Le Plessis-Robinson (FR)**
- **EVANNO, Yannick**
**c/o sanofi-aventis**
**Département brevets**
**F-75013 PARIS (FR)**
- **MALANDA, André**
**F-91140 Villejust (FR)**

(74) Mandataire: **Le Coupanec, Pascale A.M.P. et al**
**Cabinet Nony**
**3, rue de Penthièvre**
**75008 Paris (FR)**

(56) Documents cités:
**WO-A-03/068749** **WO-A-2004/072069**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 910 335 B1

## Description

L'invention a pour objet des composés dérivés de *N*-(hétéroaryl)-1-hétéroarylalkyl-1*H*-indole-2-carboxamides, qui présentent une activité antagoniste *in vitro* et *in vivo* pour les récepteurs de type TRPV1 (ou VR1).

WO 03/068749 et WO 2004/072069 décrivent des composés carboxamides qui présentent une activité antagoniste pour les recepteurs de type VR1.

Un premier objet de l'invention concerne les composés répondant à la formule générale (I) ci-après.

Un autre objet de l'invention concerne des procédés de préparation des composés de formule générale (I).

Un autre objet de l'invention concerne l'utilisation des composés de formule générale (I) notamment dans des médicaments ou dans des compositions pharmaceutiques.

Les composés de l'invention répondent à la formule générale (I) :

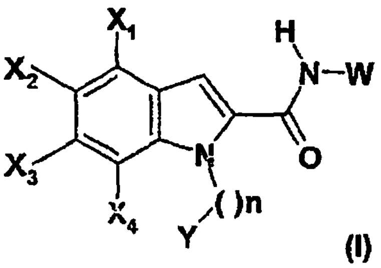

(I)

dans laquelle

| | |
|---|---|
| $X_1$, $X_2$, $X_3$, $X_4$ | représentent, indépendamment l'un de l'autre, un atome d'hydrogène ou d'halogèneou un groupe $C_1$-$C_6$-alkyle, $C_1$-$C_6$-fluoroalkyle, ou $NR_1R_2$, |
| W | représente un groupe bicyclique fusionné de formule : |

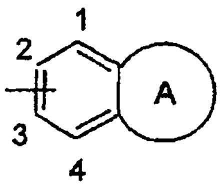

| | |
|---|---|
| | lié à l'atome d'azote par les positions 1, 2, 3 ou 4 ; |
| A | représente un hétérocycle de 5 à 7 chaînons comprenant de un à trois hétéroatomes choisi parmi O, S ou N ; le ou les atomes de carbones de A étant éventuellement substitués par un ou plusieurs groupes choisis parmi un atome d'hydrogène ou un groupe $C_1$-$C_8$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio; le ou les atomes d'azote de A étant éventuellement substitués par $R_6$ lorsque l'azote est adjacent à un atome de carbone substitué par un groupe oxo, ou par $R_7$ dans les autres cas ; |
| n | est égal à 1, 2 ou 3 ; |
| Y | représente un hétéroaryle, choisi parmi les groupes pyridinyle, pyrimidinyle, pyrazinyle, pyridazinyle, imidazolyle, benzimidazolyle, benzothiazolyle, thiazolyle, furanyle, quinoléinyle, isoquinoléinyle, quinoxalinyle, l'hétéroaryle étant éventuellement substitué par un ou plusieurs groupes choisis parmi un groupe $C_1$-$C_6$-alkyle, par exemple un méthyle, $C_1$-$C_8$-fluoroalkyle, par exemple un groupe trifluorométhyle, aryla-$C_1$-$C_8$-alkylène, par exemple un benzyle ou $NR_1R_2$ ; $R_1$ et $R_2$ formant ensemble, avec l'atome d'azote qui les porte, un groupe morpholinyle. |
| $R_1$ et $R_2$, | représentent, indépendamment l'un de l'autre, un atome d'hydrogène ou un groupe $C_1$-$C_6$-alkyle, |
| $R_6$ | représente un atome d'hydrogène ou un groupe $C_1$-$C_8$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ; |
| $R_7$ | représente un atome d'hydrogène ou un groupe $C_1$-$C_8$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_8$-alkylène, $C_1$-$C_6$-alkyle-C(O)-, $C_3$-$C_7$-cycloalkyle-$C_1$-$C_3$-alkylène-(CO)-, $C_1$-$C_8$-fluoroalkyle-C(O)-, $C_3$-$C_7$-cycloalkyle-C(O)-, aryle-C(O)-, aryle-$C_1$-$C_6$-alkyène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle. |

Dans les composés de formule générale (I) :

- le ou les atomes de soufre de l'hétérocycle A ou de l'hétéroaryle Y peuvent être sous forme oxydée (S(O) ou $S(O)_2$) ;
- le ou les atomes d'azote de l'hétérocycle A ou de l'hétéroaryle Y peuvent être sous forme oxydée (N-oxyde).

Dans le cadre de l'invention, on peut citer à titre d'exemples de groupe W, les groupes indolyle, isoindolyle, indolinyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle.

Parmi les composés de formule générale (I) objets de l'invention, un premier sous-groupe de composés est constitué par les composés pour lesquels :

$X_1$, $X_2$, $X_3$, $X_4$ représentent, indépendamment l'un de l'autre, un atome d'hydrogène ou d'halogène, par exemple un atome de fluor, ou un groupe $C_1$-$C_6$-alkyle, par exemple un groupe terbutyle, ou $C_1$-$C_6$-fluoroalkyle, par exemple un groupe trifluorométhyle.

Parmi les composés de formule générale (I) objets de l'invention, un deuxième sous-groupe de composés est constitué par les composés pour lesquels :

$X_1$, $X_2$, $X_3$, $X_4$ représentent, indépendamment l'un de l'autre, un atome d'hydrogène, d'halogène, par exemple un atome de fluor, ou un groupe $C_1$-$C_6$-alkyle, par exemple un groupe terbutyle, ou $C_1$-$C_6$-fluoroalkyle, par exemple un groupe trifluorométhyle, ou un groupe $NR_1R_2$ , $R_1$ et $R_2$ étant tels que définis dans la formule générale (I), par exemple un groupe diméthylamine.

Parmi les composés de formule générale (I) objets de l'invention, un troisième sous-groupe de composés est constitué par les composés pour lesquels :

$X_2$ est différent d'un atome d'hydrogène.

Parmi les composés de formule générale (I) objets de l'invention, un quatrième sous-groupe de composés est constitué par les composés pour lesquels :

W représente un groupe bicyclique fusionné de formule :

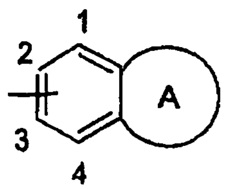

lié à l'atome d'azote par les positions 1, 2, 3 ou 4 ;
et W est choisi parmi les groupes indolinyle, isoindolyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, bonzimidozolyle, dihydrobenzimidazolyle, Indolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ;
le ou les atomes de carbone et/ou d'azote de A étant éventuellement substitués comme défini dans la formule

générale (I).

Parmi les composés du quatrième sous-groupe, un cinquième sous-groupe de composés est constitué par les composés pour lesquels:

W représente un groupe bicyclique fusionné de formule :

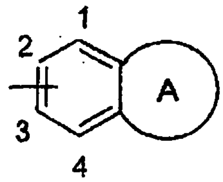

lié à l'atome d'azote par les positions 1, 2, 3 ou 4 ;
et W est choisi parmi les groupes benzimidazolyle et indolyle ; et/ou le ou les atomes de carbones de A étant éventuellement substitués par un ou plusieurs groupes $C_1$-$C_6$-alkyle, par exemple méthyle; et/ou
le ou les atomes d'azote de A étant éventuellement substitués par $R_7$, $R_7$ représentant un groupe $C_1$-$C_6$-alkyle, par exemple méthyle.

Parmi les composés du cinquième sous-groupe, un sixième sous-groupe de composés est constitué par les composés pour lesquels :

W est choisi parmi les groupes benzimidazol-5-yle et indol-5-yle ; et/ou
le ou les atomes de carbones de A étant éventuellement substitués par un ou plusieurs groupes $C_1$-$C_6$-alkyle, par exemple un méthyle ; et/ou
le ou les atomes d'azote de A étant éventuellement substitués par $R_7$, $R_7$ représentant un groupe $C_1$-$C_6$-alkyle, par exemple un méthyle.

Parmi les composés du quatrième sous-groupe, un septième sous-groupe de composés est constitué par les composés pour lesquels :

W représente un groupe bicyclique fusionné de formule :

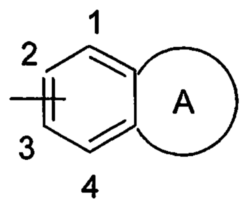

lié à l'atome d'azote par les positions 2 ou 3 ;
et W est choisi parmi les groupes benzimidazolyle, indolyle, benzothiazolyle, quinoléinyle, tétrahydroquinoléinyle et benzoxazinyle ; et/ou
le ou les atomes de carbones de A étant éventuellement substitués par un ou plusieurs groupes $C_1$-$C_6$-alkyle, par exemple un méthyle ou un isopropyle, $C_1$-$C_6$-fluoroalkyle, par exemple un groupe trifluorométhyle, $C_3$-$C_7$-cycloalkyle, par exemple un cyclopropyle, ou oxo ; et/ou
le ou les atomes d'azote de A étant éventuellement substitués par $R_6$ lorsque l'azote est adjacent à un atome de carbone substitué par un groupe oxo, $R_6$ représentant un atome d'hydrogène ou un groupe $C_1$-$C_6$-alkyle, par exemple un méthyle ; ou par $R_7$ dans les autres cas, $R_7$ représentant un groupe $C_1$-$C_6$-alkyle, par exemple un méthyle ou un groupe $C_3$-$C_7$-cycloalkyle-$C_1$-$C_3$-alkylène, par exemple un cyclopropylméthyle.

Parmi les composés de formule générale (I) objets de l'invention, un huitième sous-groupe de composés est constitué par les composés pour lesquels :

n est égal à 1 ou 2.

Parmi les composés de formule générale (I) objets de l'invention, un neuvième sous-groupe de composés est constitué par les composés pour lesquels :

Y représente un hétéroaryle, par exemple un pyridinyle, un imidazolyle, un benzimidazolyle, un thiazolyle, une quinoléinyle, l'hétéroaryle étant éventuellement substitué par un ou plusieurs groupes choisis parmi un groupe $C_1$-$C_6$-alkyle, par exemple un méthyle, $NR_1R_2$ ou aryle-$C_1$-$C_6$-alkylène, par exemple benzyle ; $R_1$ et $R_2$ formant ensemble, avec l'atome d'azote qui les porte, un groupe morpholinyle.

Parmi les composés de formule générale (I) objets de l'invention, un dixième sous-groupe de composés est constitué par les composés pour lesquels :

Y représente un hétéroaryle, choisi parmi les groupes pyridinyle, pyrimidinyle, pyrazinyle, pyridazinyle, imidazolyle, benzimidazolyle, benzothiazolyle, thiazolyle, furanyle, quinoléinyle, isoquinoléinyle, quinoxalinyle, l'hétéroaryle étant éventuellement substitué par un ou plusieurs groupes choisis parmi un groupe $C_1$-$C_6$-alkyle, par exemple un méthyle, $C_1$-$C_6$-fluoroalkyle, par exemple un groupe trifluorométhyle, aryle-$C_1$-$C_6$-alkylène, par exemple un benzyle ou $NR_1R_2$ ; $R_1$ et $R_2$ formant ensemble, avec l'atome d'azote qui les porte, un groupe morpholinyle.

Parmi les composés de formule générale (I) objets de l'invention, un onzième sous-groupe de composés est constitué par les composés pour lesquels :

W représente un groupe benzimidazolyle, le ou les atomes de carbone et/ou d'azote dudit groupe W étant éventuellement substitués comme défini dans la formule générale (I) ;
Y représente un groupe pyridinyle éventuellement substitué comme défini dans la formule générale (I).

Parmi les composés de formule générale (I) objets de l'invention, un douzième sous-groupe de composés est constitué par les composés pour lesquels :

$X_1$, $X_2$, $X_3$, $X_4$ représentent, indépendamment l'un de l'autre, un atome d'hydrogène ou d'halogène, par exemple un atome de fluor, ou un groupe $C_1$-$C_6$-alkyle, par exemple un groupe terbutyle, $C_1$-$C_6$-fluoroalkyle, par exemple un groupe trifluorométhyle, ou $NR_1R_2$, $R_1$ et $R_2$ étant tels que définis dans la formule générale (I), par exemple un groupe diméthylamine ;

W représente un groupe bicyclique fusionné de formule :

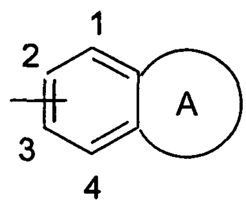

lié à l'atome d'azote par les positions 2 ou 3 ; et W est choisi parmi les groupes benzimidazolyle, indolyle, benzothiazolyle, quinoléinyle, tétrahydroquinoléinyle, benzoxazinyle ;
le ou les atomes de carbones de A étant éventuellement substitués par un ou plusieurs groupes $C_1$-$C_6$-alkyle, par exemple méthyle ou isopropyle, $C_1$-$C_6$-fluoroalkyle, par exemple un groupe trifluorométhyle, $C_3$-$C_7$-cycloalkyle, par exemple cyclopropyle, ou oxo ; et/ou
le ou les atomes d'azote de A étant éventuellement substitués par $R_6$ lorsque l'azote est adjacent à un atome de carbone substitué par un groupe oxo, $R_6$ représentant un atome d'hydrogène ou un groupe $C_1$-$C_6$-alkyle, par exemple un méthyle ; ou par $R_7$ dans les autres cas, $R_7$ représentant un groupe $C_1$-$C_6$-alkyle, par exemple un méthyle ou un groupe $C_3$-$C_7$-cycloalkyle-$C_1$-$C_3$-alkylène, par exemple un cyclopropylméthyle ; et/ou

n est égal à 1 ou 2 ; et/ou

Y représente un hétéroaryle, choisi parmi les groupes pyridinyle, pyrimidinyle, pyrazinyle, pyridazinyle, imidazolyle, benzimidazolyle, benzothiazolyle, thiazolyle, furanyle, quinoléinyle, isoquinoléinyle, quinoxalinyle, l'hétéroaryle étant éventuellement substitué par un ou plusieurs groupes choisis parmi un groupe $C_1$-$C_6$-alkyle, par exemple un méthyle, $C_1$-$C_6$-fluoroalkyle, par exemple un groupe trifluorométhyle, aryle-$C_1$-$C_6$-alkylène, par exemple un benzyle ou $NR_1R_2$ ; $R_1$ et $R_2$ formant ensemble, avec l'atome d'azote qui les porte, un groupe morpholinyle.

Parmi les composés de formule générale (I) objets de l'invention, un treizième sous-groupe de composés est constitué par les composés pour lesquels :

- $X_1$ et $X_4$ représentent chacun un atome d'hydrogène ;
- $X_2$ et $X_3$ sont tels que l'un des deux représente un atome d'hydrogène alors que l'autre représente un groupe choisi parmi un atome d'halogène, par exemple un atome de fluor, ou un groupe $C_1$-$C_6$-alkyle, par exemple un groupe terbutyle, $C_1$-$C_6$-fluoroalkyle, par exemple un groupe trifluorométhyle, ou $NR_1R_2$, $R_1$ et $R_2$ étant tels que définis dans la formule générale (I), , par exemple un groupe diméthylamine ;
- W représente un groupe bicyclique fusionné de formule :

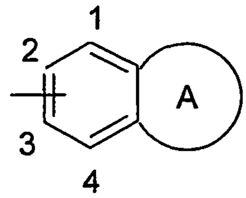

lié à l'atome d'azote par les positions 2 ou 3 ; et W est choisi parmi les groupes benzimidazolyle, benzothiazolyle, quinoléinyle, tétrahydroquinoléinyle ;
les atomes de carbones de A étant éventuellement substitués par un ou plusieurs groupes $C_1$-$C_6$-alkyle, par exemple méthyle ; et/ou
le ou les atomes d'azote de A étant éventuellement substitués par $R_7$, $R_7$ représentant un groupe $C_1$-$C_6$-alkyle, par exemple méthyle ;
- n est égal à 1
- Y représente un hétéroaryle, par exemple un groupe pyridinyle, pyrazinyle, benzothiazolyle, quinoléinyle, isoquinoléinyle, quinoxaléinyle ; l'hétéroaryle étant éventuellement substitué par un ou plusieurs groupes $C_1$-$C_6$-alkyle, par exemple un méthyle ou $NR_1R_2$ ; $R_1$ et $R_2$ formant ensemble, avec l'atome d'azote qui les porte, un groupe morpholinyle.

Les composés pour lesquels à la fois $X_1$, $X_2$, $X_3$, $X_4$, W, n et Y sont tels que définis dans les sous-groupes de composés ci-dessus, forment un quatorzième sous-groupe.

Dans le cadre de la présente invention on entend par :

- $C_t$-$C_z$ où t et z peuvent prendre les valeurs de 1 à 7, une chaîne carbonée pouvant avoir de t à z atomes de carbone, par exemple $C_1$-$C_3$ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe $C_{1-3}$-alkylène représente une chaîne carbonée divalente de 1 à 3 atomes de carbone, linéaire ou ramifiée, par exemple un méthylène, éthylène, 1-méthyléthylène, propylène ;
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphtyle ;
- un hétéroaryle : un groupe cyclique aromatique de 5 à 10 chaînons contenant de 1 à 4 hétéroatomes choisis parmi O, S ou N. A titre d'exemple, on peut citer les groupes imidazolyle, thiazolyle, oxazolyle, furanyle, thiophényle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, benzothiazolyle, isobenzothiazolyle, benzotriazolyle, quinoléinyle, isoquinoléinyle, quinoxalinyle ;
- un hétérocycle : un groupe cyclique saturé, partiellement insaturé ou aromatique de 5 à 7 chaînons, comprenant de un à trois hétéroatomes choisis parmi O, S ou N ;
- un atome d'halogène : un fluor, un chlore, un brome ou un iode ;

- « oxo » signifie « =O » ;
- « thio » signifie « =S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvates, à savoir sous forme d'associations ou de combinaisons avec une ou plusieurs molécules d'eau ou avec un solvante. De tels hydrates et solvates font également partie de l'invention.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.

Schéma 1

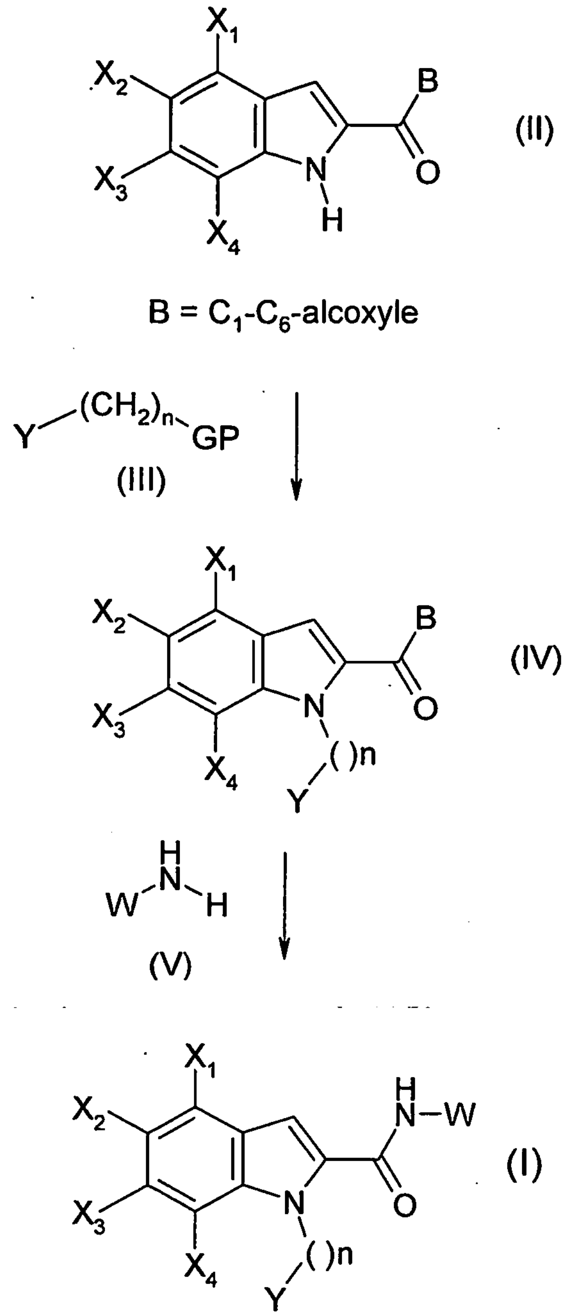


Selon le schéma 1, les composés de formule générale (IV) peuvent être obtenus par réaction d'un composé de formule générale (II) dans laquelle $X_1$, $X_2$, $X_3$, $X_4$ sont tels que définis dans la formule générale (I) et B représente un groupe $C_1$-$C_6$-alcoxyle, avec un composé de formule générale (III), dans laquelle Y et n sont tels que définis dans la formule générale (I) et GP représente un groupe partant ou GP représente un groupe hydroxyle.

Les composés de formule générale (II) sont disponibles dans le commerce ou préparés selon de nombreux procédés décrits dans la littérature (D. Knittel Synthesis 1985, 2, 186 ; T.M. Williams J.Med.Chem. 1993, 36 (9), 1291 ; JP2001151771A2 par exemple).

Lorsque le composé de formule générale (III), est défini tel que n est égal à 1, 2 ou 3 et GP représente un groupe partant tel qu'un atome de chlore, de brome ou d'iode, la réaction peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1), 1).

Lorsque le composé de formule générale (III) est défini tel que n est égal à 1, 2 ou 3 et GP représente un groupe hydroxyle, les composés de formule générale (IV), peuvent être obtenus par réaction du composé de formule générale (II) avec un composé de formule générale (III) en présence d'une phosphine telle que, par exemple, la triphénylphosphine et d'un réactif tel que, par exemple, l'azodicarboxylate de diéthyle en solution dans un solvant tel que le dichlorométhane ou le tétrahydrofuranne (O. Mitsonobu, Synthesis, 1981, 1-28).

Le composé de formule générale (I) est ensuite obtenu par réaction d'un composé de formule générale (IV), tel qu'obtenu ci-dessus, avec un amidure du composé de formule générale (V), dans laquelle W est tel que défini dans la formule générale (I), au reflux d'un solvant tel que le toluène. L'amidure du composé de formule générale (V) est préparé par action préalable du triméthylaluminium sur les amines de formule générale (V).

Les composés de formules générales (I), (II) et (IV), dans lesquelles $X_1$, $X_2$, $X_3$ et/ou $X_4$, représentent un groupe $NR_1R_2$, peuvent être obtenus à partir des composés de formules générales(I), (II) et (IV) correspondants, dans lesquelles $X_1$, $X_2$, $X_3$, et/ou $X_4$, représente un groupe nitro, par exemple par réduction, puis acylation ou sulfonylation, selon des méthodes qui sont décrites dans la littérature ou qui sont connues de l'homme du métier.

Les composés de formules générales (I), (II) et (IV), dans lesquelles $X_1$, $X_2$, $X_3$ et/ou $X_4$, représentent un groupe $NR_1R_2$, peuvent être obtenus à partir des composés de formules générales (I), (II) et (IV) correspondants, dans lesquelles $X_1$, $X_2$, $X_3$, et/ou $X_4$, représente, par exemple, un atome de brome par réaction de couplage respectivement avec une amine, un amide ou une sulfonamide en présence d'une base, d'une phosphine et d'un catalyseur à base de palladium, selon des méthodes qui sont décrites dans la littérature ou qui sont connues de l'homme du métier.

Les composés de formule générale (I), dans laquelle $R_7$ représente un atome d'hydrogène, peuvent être obtenus à partir des composés de formule générale (I) dans laquelle, par exemple, $R_7$ représente un groupe phénylméthyle, par hydrogénation catalysée, par le palladium par exemple, ou par toutes méthodes qui sont décrites dans la littérature ou qui sont connues de l'homme du métier.

Dans ce qui précède, les composés de formule (III) sont disponibles dans le commerce, décrits dans la littérature (Carling R.W. et al J.Med.Chem. 2004 (47), 1807 - 1822 ou Russel M.G.N. et al. J.Med.Chem. 2005 (48), 1367 - 1383) ou accessibles en utilisant des méthodes connues de l'homme du métier. Les composés (V) et les autres réactifs, quand leur mode de préparation n'est pas décrit, sont disponibles dans le commerce ou décrits dans la littérature (WO03049702, WO03068749 par exemple).

L'invention, selon un autre de ses aspects, a également pour objet des composés de formule ($IV_{1-35}$) ou ($V_{1-8}$). Ces composés sont utiles comme intermédiaires de synthèse des composés de formule (I) et, de façon plus générale, dans la préparation de composés à visée thérapeutique.

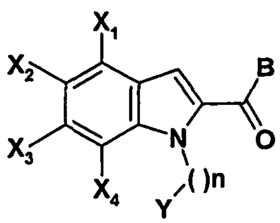
($IV_{1-35}$)

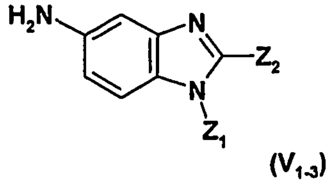
($V_{1-3}$)

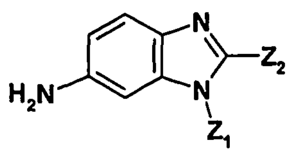
($V_{4-8}$)

Les indoles ($IV_{1-35}$), listés dans le tableau 1, ont tous été préparés selon l'une des méthodes décrites dans le schéma 1.

Le tableau 1 qui suit illustre les structures chimiques et les propriétés physiques de quelques composés de formule générale ($IV_{1-35}$) selon l'invention. Dans ce tableau :

- La colonne "PF" renseigne les points de fusion des produits en degrés Celsius (°C) ;
- Lorsque les produits ont été isolés sous la forme de solide amorphe ou d'huile, ils sont caractérisés dans cette colonne par leur masse ($[MH]^+$) ou leurs données RMN (RMN) détaillées ci-après ;
- t-Bu représente un groupe *tertio*-butyle, Me un groupe méthyle et Et un groupe éthyle.

Tableau 1

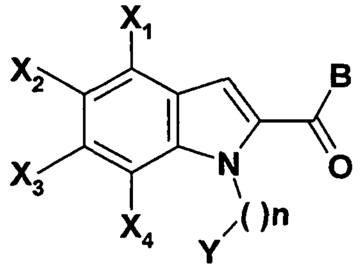

($IV_{1-35}$)

| N° | $X_1$, $X_2$, $X_3$, $X_4$ | n | Y | B | PF (°C) |
|---|---|---|---|---|---|
| $IV_1$ | H, F, H, H | 1 | thiazol-2-yle | OEt | 95 - 96 |
| $IV_2$ | H, F, H, H | 2 | pyridin-3-yle | OEt | 105 - 106 |
| $IV_3$ | H, F, H, H | 1 | pyridin-3-yle | OEt | 104 - 105 |
| $IV_4$ | H, F, H, H | 1 | pyridin-4-yle | OEt | RMN |
| $IV_5$ | H, F, H, H | 1 | pyridin-2-yle | OEt | 67 - 69 |
| $IV_6$ | H, F, H, H | 1 | 2-méthyl-pyridin-3-yle | OEt | $[MH]^+$ = 313 |
| $IV_7$ | H, F, H, H | 1 | 1-N-benzyl-imidazol-2-yle | OEt | 84 - 85 |
| $IV_8$ | H, F, H, H | 1 | 2-pyrrolidino-pyridin-3-yle | OEt | $[MH]^+$ = 368 |
| $IV_9$ | H, F, H, H | 1 | 2-benzothiazol-2-yle | OEt | $[MH]^+$ = 355 |
| $IV_{10}$ | H, F, H, H | 1 | 1-méthyl-benzimidazol-2-yle | OEt | 198 - 199 |
| $IV_{11}$ | H, F, H, H | 2 | 4-méthyl-thiazol-5-yle | OEt | $[MH]^+$ = 333 |
| $IV_{12}$ | H, F, H, H | 1 | Quinoléin-2-yle | OEt | 105 - 106 |
| $IV_{13}$ | H, F, H, H | 1 | Quinoxalin-2-yle | OEt | $[MH]^+$ = 350 |
| $IV_{14}$ | H, F, H, H | 1 | Pyrazin-2-yle | OEt | $[MH]^+$ = 300 |
| $IV_{15}$ | H, F, H, H | 1 | 3-méthyl-pyridin-2-yle | OEt | $[MH]^+$ = 313 |
| $IV_{16}$ | H, F, H, H | 1 | Isoquinolin-1-yle | OEt | 125-126 |
| $IV_{17}$ | H, F, H, H | 1 | 4-méthyl-pyridin-2-yle | OEt | $[MH]^+$ = 313 |
| $IV_{18}$ | H, F, H, H | 2 | 2-méthyl-pyridin-4-yle | OEt | $[MH]^+$ = 327 |
| $IV_{19}$ | H, F, H, H | 1 | 2-méthyl-pyridin-4-yle | OEt | 71 - 72 |
| $IV_{20}$ | H, F, H, H | 1 | 5-méthyl-pyridin-2-yle | OEt | $[MH]^+$ = 313 |
| $IV_{21*}$ | H, F, H, H | 1 | diméthyl-pyrazin-2-yle* | OEt | $[MH]^+$ = 328* |
| $IV_{22}$ | H, F, H, H | 1 | 6-méthyl-pyrazin-2-yle | OEt | $[MH]^+$ = 314 |
| $IV_{23}$ | H, F, H, H | 1 | 6-méthyl-pyridazin-3-yle | OEt | $[MH]^+$ = 314 |
| $IV_{24}$ | H, F, H, H | 1 | 6-méthyl-pyridin-2-yle | OEt | RMN |
| $IV_{25}$ | H, F, H, H | 1 | 2-phényl-pyridin-4-yle | OEt | RMN |
| $IV_{26}$ | H, H, tBu, H | 1 | pyridin-4-yle | OMe | RMN |
| $IV_{27}$ | H, $CF_3$, H, H | 1 | pyridin-4-yle | OEt | RMN |
| $IV_{28}$ | H, H, $NMe_2$, H | 1 | pyridin-4-yle | OEt | RMN |
| $IV_{29}$ | H, H, $CF_3$, H | 1 | pyridin-4-yle | OMe | RMN |
| $IV_{30}$ | H, tBu, H, H | 1 | pyridin-4-yle | OEt | RMN |

(suite)

| N° | $X_1$, $X_2$, $X_3$, $X_4$ | n | Y | B | PF (°C) |
|---|---|---|---|---|---|
| $IV_{31}$ | H, tBu, H, H | 1 | pyridin-2-yle | OEt | RMN |
| $IV_{32}$ | H, tBu, H, H | 1 | pyridin-3-yle | OEt | RMN |
| $IV_{33}$ | H, tBu, H, H | 1 | 2-méthyl-pyridin-3-yle | OEt | $[MH]^+ = 351$ |
| $IV_{34}$ | H, tBu, H, H | 1 | 6-méthyl-pyridin-2-yle | OEt | $[MH]^+ = 351$ |
| $IV_{35}$ | H, F, H, H | 1 | pyrimidin-4-yle | OEt | $[MH]^+ = 300$ |
| *Le produit $IV_{21}$ a été obtenu sous la forme d'un mélange de 3 isomères engagé, tel quel, dans la suite de la synthèse de (I), décrite dans le schéma 1, pour conduire après séparation par chromatographie aux produits 94, 95 et 96 (voir partie expérimentale). | | | | | |

Les RMN de quelques composés du tableau 1 sont présentées ci-dessous :

Composé n° $IV_4$: R.M.N. $^1H$ ($CDCl_3$), $\delta$ (ppm): 1,3 (t, 3H) ; 4,2 (q, 2H) ; 5,79 (s, 2H) ; 6,82 (d, 2H) ; 7,07 (m, 2H) ; 7,27 (m, 2H) ; 8,41 (d, 2H).

Composé n° $IV_{24}$: R.M.N. $^1H$ ($CDCl_3$), $\delta$ (ppm):1,38 (t, 3H) ; 2,6 (s, 3H) ; 4,32 (q, 2H) ; 5,91 (s, 2H) ; 6,39 (d, 1 H) ; 7,05 (m, 2H) ; 7,35 (m, 4H).

Composé n° $IV_{25}$: R.M.N. $^1H$ ($CDCl_3$), $\delta$ (ppm):1,54 (t, 3H) ; 4,51 (q, 2H) ; 6,1 (s, 2H) ; 6,97 (m, 1 H) ; 7,29 (txd, 1 H) ; 7,41 (m, 1 H) ; 7,6 (m, 6H) ; 8,06 (m, 2H) ; 7,5 (d, 1 H).

Composé n° $IV_{26}$: R.M.N. $^1H$ (DMSO $D_6$), $\delta$ (ppm): 1,25 (s, 9H) ; 3,79 (s, 3H) ; 5,90 (s, 2H) ; 6,96 (dxd, 2H) ; 7,27 (dxd, 1 H) ; 7,35 (s, 1 H) ; 7,46 (s, 1 H) ; 7,66 (d, 1 H) ; 8,45 (dxd, 2H).

Composé n° $IV_{27}$: R.M.N. $^1H$ (DMSO $D_6$), $\delta$ (ppm): 1,27 (t, 3H) ; 4,29 (q, 2H) ; 5,95 (s, 2H) ; 6,93 (d, 2H) ; 7,58 (s, 1 H) ; 7,61 (dxd, 1 H) ; 7,80 (d, 1 H) ; 8,22 (s, 1 H) ; 8,45 (dxd, 2H).

Composé n° $IV_{28}$: R.M.N. $^1H$ (DMSO $D_6$), $\delta$ (ppm): 1,23 (t, 3H) ; 2,91 (s, 6H) ; 4,2 (q, 2H) ; 5,79 (s, 2H) ; 6,56 (s, 1H) ; 6,8 (dxd, 1H) ; 6,94 (dxd, 2H) ; 7,26 (s, 1H) ; 7,52 (d, 1H) ; 8,45 (dxd, 2H).

Composé n° $IV_{29}$: R.M.N. $^1H$ (DMSO $D_6$), $\delta$ (ppm): 3,82 (s, 3H) ; 6 (s, 2H) ; 6,9 (d, 2H) ; 7,46 (d, 1H) ; 7,54 (s, 1 H) ; 7,99 (d, 1 H) ; 8,08 (s, 1 H) ; 8,45 (dxd, 2H).

Composé n° $IV_{30}$: R.M.N. $^1H$ ($CDCl_3$), $\delta$ (ppm): 1,41 (m, 12H) ; 4,35 (q, 2H) ; 5,81 (s, 2H) ; 6,97 (d, 2H) ; 7,21 (d, 1 H) ; 7,45 (m, 2H) ; 7,74 (m, 1 H) ; 8,54 (d, 2H).

Composé n° $IV_{31}$: R.M.N. $^1H$ (DMSO $D_6$), $\delta$ (ppm): 1,25 (t, 3H) ; 1,32 (s, 9H) ; 4,25 (q, 2H) ; 5,88 (s, 2H) ; 6,79 (d, 1 H) ; 7,22 (m, 1 H) ; 7,32 (s, 1 H) ; 7,4 (m, 2H) ; 7,65 (m, 2H) ; 8,46 (m, 1 H).

Composé n° $IV_{32}$: R.M.N. $^1H$ (DMSO $D_6$), $\delta$ (ppm): 1,28 (t, 3H) ; 1,32 (s, 9H) ; 4,29 (q, 2H) ; 5,85 (s, 2H) ; 7,27 (m, 1 H) ; 7,35 (m, 2H) ; 7,48 (m, 2H) ; 7,65 (d, 1 H) ; 8,36 (d, 1H) ; 8,41 (d, 1 H).

Les amines ($V_{1-6}$), peuvent être préparées selon la voie de synthèse décrite dans le schéma 2.

Dans ce schéma, $Z_1$ et $Z_2$ représentent chacun indépendamment l'un de l'autre un groupe $C_1$-$C_6$-alkyle, $C_3$-$C_7$-cycloalkyle ou $C_3$-$C_7$-cycloalkyle- $C_1$-$C_3$-alkylène.

La cyclisation mettant en jeu la 4-nitro-1,2-phenylènediamine (VI) et un réactif tel qu'un acide carboxylique de formule $Z_2$-$CO_2H$ dans laquelle $Z_2$ représente un groupe $C_1$-$C_6$-alkyle, $C_3$-$C_7$-cycloalkyle ou $C_3$-$C_7$-cycloalkyle-$C_1$-$C_3$-alkylène, permet la formation du benzimidazole (VII). Celui-ci peut ensuite être substitué par un groupe Z1, par réaction avec un composé de formule $Z_1$-GP, où GP défini tel que dans le schéma 1 et Z1 représente un groupe $C_1$-$C_6$-alkyle, $C_3$-$C_7$-cycloalkyle ou $C_3$-$C_7$-cycloalkyle- $C_1$-$C_3$-alkylène, par exemple en présence d'une base telle que l'hydrure de sodium dans un solvant comme le tétrahydrofuranne. Le mélange de benzimidazoles (VIII) résultant est ensuite transformé en amines ($V_{1-6}$) par réduction, par exemple, par hydrogénation catalytique en présence d'un catalyseur tel que le palladium sur charbon ou selon toute autre méthode de réduction d'un groupe nitro en amine, connue

de l'homme du métier.

Schéma 2

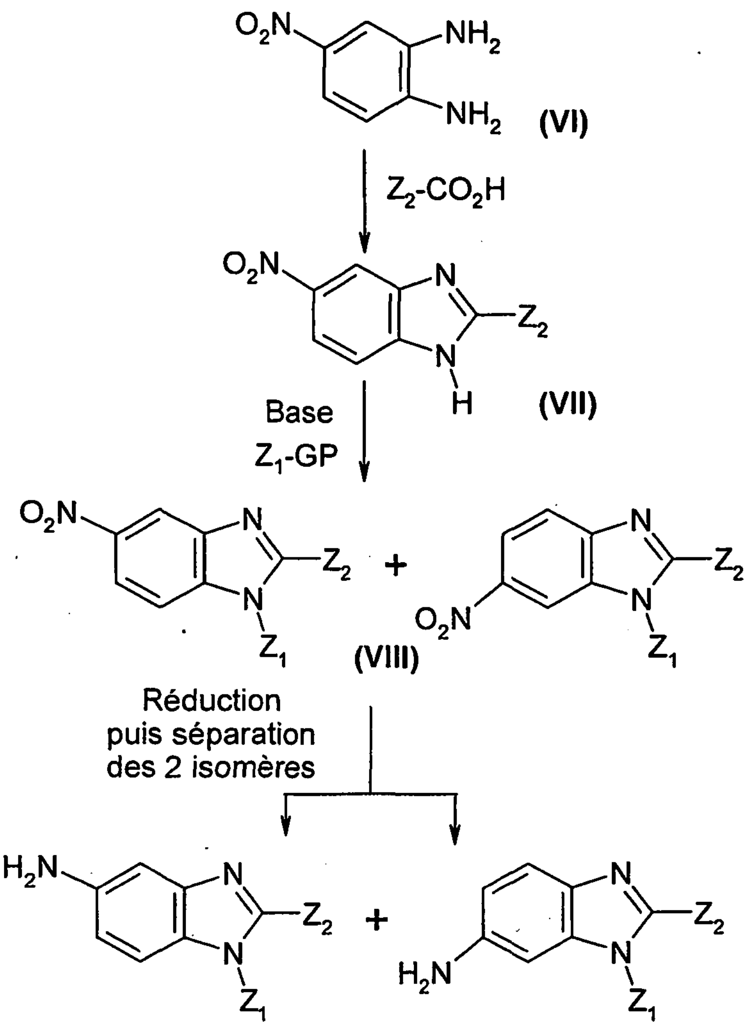


($V_{1-3}$) ($V_{4-6}$)

Les amines ($V_{1-6}$) sont listées dans les tableaux 2a et 2b. La description du mode de préparation de l'une de ces amines est détaillée en partie expérimentale.

Tableau 2a

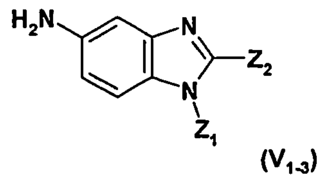

($V_{1-3}$)

| N° | $Z_1$ | $Z_2$ | Masse $[MH]^+$ |
|---|---|---|---|
| $V_1$ | (cyclopropyl)méthyle | méthyle | 202 |
| $V_2$ | méthyle | cyclopropyle | 188 |
| $V_3$ | méthyle | isopropyle | 190 |

Tableau 2b

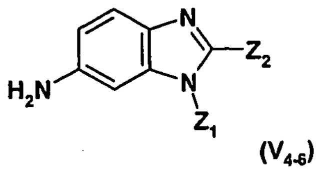

(V4-6)

| N° | $Z_1$ | $Z_2$ | Masse $[MH]^+$ |
|---|---|---|---|
| $V_4$ | (cyclopropyl)méthyle | méthyle | 202 |
| $V_5$ | méthyle | cyclopropyle | 188 |
| $V_6$ | méthyle | isopropyle | 190 |

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 3. Les microanalyses élémentaires, les analyses LC-MS (chromatographie liquide couplée à la spectrométrie de masse) les spectres I.R. ou les spectres R.M.N. confirment les structures des composés obtenus.

**Exemple 1 (Composé N°3)**

Chlorhydrate (1 :1) de *N*-(1-méthyl-1*H*-indol-5-yl)-5-fluoro-1-[(pyridin-4-yl)méthyl]-1*H*-indole-2-carboxamide

1.1. 5-fluoro-1-[(pyridin-4-yl)méthyl]-1*H*-indole-2-carboxylate d'éthyle

On ajoute, goutte à goutte, une solution de 1 g (4,73 mmoles) de 5-fluoro-1*H*-indole-2-carboxylate d'éthyle sur une suspension de 0,38 g (9,45 mmoles) d'hydrure de sodium à 60% dans 10 mL de diméthylformamide, agitée à 0°C sous argon. Le mélange est agité 30 minutes à 0°C puis 30 min à 20°C. On refroidit le mélange réactionnel et on ajoute en plusieurs portions 1,24 g (4,2 mmoles) de bromhydrate de 4-bromométhylpyridine. Le mélange est agité 30 min à 0°C puis 30 min à 20°C. On refroidit à nouveau le mélange réactionnel à 0°C et on ajoute 0,38 g supplémentaire (9,45 mmoles) d'hydrure de sodium à 60% dans 10 mL de diméthylformamide. Après 30 min à 0°C, on ajoute, en plusieurs portions, 1,24 g (4,2 mmoles) de bromhydrate de 4-bromométhylpyridine. Le mélange réactionnel est ensuite agité 20 h à 20°C. Après ce temps le mélange est versé sur une solution de 100 mL d'eau glacée et 100 mL d'éther éthylique. La phase organique est séparée et la phase aqueuse réextraite avec 100 mL d'éther éthylique. Les phases organiques sont réunies, lavées avec 50 mL d'eau puis séchées sur sulfate de sodium, filtrées puis concentrées sous pression réduite. On purifie le résidu par chromatographie préparative (éluant : dichlorométhane - acétone). On obtient 0,65 g de produit attendu sous la forme d'une huile utilisée telle quelle dans la suite de la synthèse.

1.2 Chlorhydrate (1:1) de *N*-(1-méthyl-1*H*-indol-5-yl)-5-fluoro-1-[(pyridin-4-yl)méthyl]-1*H*-indole-2-carboxamide (composé n°3)

On ajoute sous argon, à une solution de 0,18 g (1,21 mmole) de 5-amino-1-méthyl-1*H*-indole (I.T. Forbes, J.Med.Chem. 1993, 36 (8), 1104) dans 10 mL de toluène sec, 1,26 mL de triméthylaluminium (2M dans le toluène). Après 15 min, on ajoute 0,3 g (1,01 mmole) de 5-fluoro-1-[(pyridin-4-yl)méthyl]-1*H*-indole-2-carboxylate d'éthyle, obtenu à l'étape 1.1. On porte à reflux pendant 4 h puis on agite le milieu réactionnel à température ambiante toute la nuit. On le verse sur de la glace et on extrait par deux fois 30 mL de dichlorométhane. Les phases organiques sont réunies, lavées avec 50 mL d'eau puis séchées sur sulfate de sodium, filtrées puis concentrées sous pression réduite. On purifie le résidu par chromatographie préparative (éluant : dichlorométhane - acétone). On obtient 0,35 g d'un solide qui est séché sous pression réduite.
Point de fusion (base) : 204 - 205 °C
Le solide résultant est repris dans 30 mL de dichlorométhane et 0,26 mL d'une solution d'acide chlorhydrique 4N dans le dioxane est ajouté. La solution est concentrée sous pression réduite et le solide résultant recristallisé dans un mélange d'isopropanol et de méthanol. On obtient ainsi 0,33 g du produit attendu sous la forme d'un chlorhydrate.
Point de fusion (1 HCl) : 258 - 260 °C
R.M.N. $^1H$ (DMSO $D_6$), $\delta$ (ppm): 3,76 (s, 3H) ; 6,1 (s, 2H) ; 6,33 (d, 1H) ; 7,11 (dxd, 1H) ; 7,25 (d, 2H) ; 7,37 (m, 2H) ; 7,52 (m, 5H) ; 7,9 (s, 1H) ; 8,7 (d, 2H).

**<u>Exemple 2</u> (Composé N°4)**

Chlorhydrate (2 :1) de *N*-(1-méthyl-1*H*-benzimidazol-5-yl)-5-fluoro-1-[(pyridin-3-yl)méthyl]-1*H*-indole-2-carboxamide

2.1. 5-fluoro-1-[(pyridin-3-yl)méthyl]-1*H*-indole-2-carboxylate d'éthyle

On ajoute, goutte à goutte, une solution de 1 g (4,73 mmoles) de 5-fluoro-1*H*-indole-2-carboxylate d'éthyle sur une suspension de 0,38 g (9,45 mmoles) d'hydrure de sodium à 60% dans 10 mL de diméthylformamide, agitée à 0°C sous argon. Le mélange est agité 30 min à 0°C puis 30 min à 20°C. On refroidit le mélange réactionnel et on ajoute en plusieurs portions 1.24 g (4,8 mmoles) de bromhydrate de 3-bromométhylpyridine. Le mélange est agité 30 min à 0°C puis 30 min à 20°C. On refroidit à nouveau le mélange réactionnel à 0°C et on ajoute 0,38 g supplémentaire (9,45 mmoles) d'hydrure de sodium à 60% dans 10 mL de diméthylformamide. Après 30 min à 0°C, on ajoute, en plusieurs portions, 1,24 g (4,8 mmoles) de bromhydrate de 3-bromométhylpyridine. Le mélange réactionnel est agité 58 h à 20°C. Le mélange est ensuite versé sur une solution de 100 mL d'eau glacée et 100 mL d'éther éthylique. La phase organique est séparée et la phase aqueuse réextraite avec 100 mL d'éther éthylique. Les phases organiques sont réunies, lavées avec 50 m d'eau puis séchées sur sulfate de sodium, filtrées puis concentrées sous pression réduite. On purifie le résidu par chromatographie préparative (éluant : dichlorométhane - acétone). On obtient 0,5 g de produit attendu sous la forme d'un solide qui est utilisé tel quel dans la suite de la synthèse.
PF = 104 -105°C

2.2 Chlorhydrate (2:1) de *N*-(1-méthyl-1*H*-benzimidazol-5-yl)-5-fluoro-1-[(pyridin-3-yl)méthyl]-1*H*-indole-2-carboxamide (composé n°4)

On ajoute sous argon, à une solution de 0,414 g (2,82 mmoles) de 5-amino-1-méthyl-1*H*-benzimidazole dans 10 mL de toluène sec, 4,1 mL de triméthylaluminium (2M dans le toluène). Après 15 min, on ajoute 0,7 g (2,35 mmoles) de 5-fluoro-1-[(pyridin-3-yl)méthyl]-1*H*-indole-2-carboxylate d'éthyle, obtenu à l'étape 2.1. On porte à reflux pendant 4 h puis on agite le milieu réactionnel à température ambiante toute la nuit. On le verse sur 100 g de glace et 50 mL de dichlorométhane. On obtient une suspension que l'on filtre et qu'on lave à l'eau et à l'éther. On purifie le résidu par chromatographie préparative sur alumine (éluant : dichlorométhane - méthanol). On obtient 0,36 g d'un solide qui est séché sous pression réduite.

Le solide résultant est repris dans 30 mL de dichlorométhane et 0,55 mL d'une solution d'acide chlorhydrique 4N dans le dioxane est ajouté. La solution est concentrée sous pression réduite et le solide résultant recristallisé dans un mélange d'isopropanol et de méthanol. On obtient ainsi 0,36 g du produit attendu sous la forme d'un chlorhydrate.
Point de fusion (2HCl) : 268 - 270 °C
R.M.N. $^1$H (DMSO $D_6$), $\delta$ (ppm): 4,03 (s, 3H) ; 6 (s, 2H) ; 7,18 (dxd, 1 H) ; 7,56 (dxd, 1H) ; 7,68 (m, 2H) ; 7,9 (m, 4H) ; 8,41 (s, 1H); 8,69 (m, 2H) ; 9,59 (s, 1H).

**<u>Exemple 3</u> Composé N°6)**

Chlorhydrate (2:1) de *N*-(1,2-diméthyl-1*H*-benzimidazol-5-yl)-5-*t*-butyl-1-[(pyridin-4-yl)méthyl]-1*H*-indole-2-carboxamide

3.1. 5-*t*-butyl-1-[(pyridin-4-yl)méthyl]-1*H*-indole-2-carboxylate d'éthyle

On ajoute, goutte à goutte, une solution de 1 g (4,08 mmoles) de 5-*t*-butyl-1*H*-indole-2-carboxylate d'éthyle sur une suspension de 0,33 g (8,15 mmoles) d'hydrure de sodium à 60% dans 10 mL de diméthylformamide, agitée à 0°C sous argon. Le mélange est agité 30 min à 0°C puis 30 min à 20°C. On refroidit le mélange réactionnel et on ajoute en plusieurs portions 1,06 g (4,08 mmoles) de bromhydrate de 4-bromométhylpyridine. Le mélange est agité 30 min à 0°C puis 30 min à 20°C. On refroidit à nouveau le mélange réactionnel à 0°C et on ajoute 0,33 g supplémentaire (8,15 mmoles) d'hydrure de sodium à 60% dans 10 mL de diméthylformamide. Après 30 min à 0°C, on ajoute, en plusieurs portions, 1,06 g (4,08 mmoles) de bromhydrate de 4-bromométhylpyridine. Le mélange réactionnel est ensuite agité 20 h à 20°C. Après ce temps le mélange est versé sur une solution de 100 mL d'eau glacée et 70 mL d'éther éthylique. La phase organique est séparée et la phase aqueuse réextraite avec 50 mL d'éther éthylique. Les phases organiques sont réunies, lavées avec 50 mL d'eau puis séchées sur sulfate de sodium, filtrées puis concentrées sous pression réduite. On purifie le résidu par chromatographie préparative (éluant : dichlorométhane - acétone). On obtient 0,7 g de produit attendu sous la forme d'une huile utilisée telle quelle dans la suite de la synthèse.

3.2 Chlorhydrate (2:1) de *N*-(1,2-diméthyl-1*H*-benzimidazol-5-yl)-5-*t*-butyl-1-[(pyridin-4-yl)méthyl]-1*H*-indole-2-carboxamide (composé n°6)

On ajoute sous argon, à une solution de 0,24 g (1,43 mmole) de 5-amino-1,2-diméthyl-1*H*-benzimidazole (WO2002059110) dans 20 mL de toluène sec, 0,9 mL de triméthylaluminium (2M dans le toluène). Après 15 min, on ajoute 0,4 g (1,19 mmole) de 5-*t*-butyl-1-[(pyridin-4-yl)méthyl]-1*H*-indole-2-carboxylate d'éthyle, obtenu à l'étape 3.1. On porte à reflux pendant 4 h puis on agite le milieu réactionnel à température ambiante toute la nuit. On le verse sur 150 g de glace et 70 mL de dichlorométhane. On sépare la phase aqueuse que l'on extrait par deux fois 30 mL de dichlorométhane. Les phases organiques sont réunies, lavées avec 50 mL d'eau puis séchées sur sulfate de sodium, filtrées puis concentrées sous pression réduite. On purifie le résidu par chromatographie préparative (éluant : dichlorométhane - méthanol). On obtient 0,4 g d'un solide qui est séché sous pression réduite
PF (base) : 270 - 272°C
Le solide résultant est repris dans 30 mL d'un mélange 9/1 de dichlorométhane et de méthanol et 0,5 mL d'une solution d'acide chlorhydrique 4N dans le dioxane est ajouté. La solution est concentrée sous pression réduite et le solide résultant recristallisé dans un mélange d'éthanol et d'eau. On obtient ainsi 0,22 g du produit attendu sous la forme d'un chlorhydrate.
Point de fusion (2HCl): 295 - 300 °C
R.M.N. $^1$H (DMSO $D_6$), $\delta$ (ppm): 1,31 (s, 9H), 2,79 (s, 3H), 3,89 (s, 3H) ; 6,08 (s, 2H); 7,42 (m, 4H) ; 7,8 (m, 4H) ; 8,3 (s, 1 H) ; 8,7 (d, 2H) ; 10,9 (s, 1 H échangeable).

**Exemple 4 (Composé N°7)**

Chlorhydrate (2:1) de *N*-(1,2-diméthyl-1*H*-benzimidazol-5-yl)-5-fluoro-1-[(pyridin-4-yl)méthyl]-1*H*-indole-2-carboxamide (Composé n°7)

On ajoute sous argon, à une solution de 0,27 g (1,61 mmole) de 5-amino-1,2-diméthyl-1H-benzimidazole (WO02059110) dans 20 mL de toluène sec, 1 mL de triméthylaluminium (2M dans le toluène). Après 15 min, on ajoute 0,4 g (1, 34 mmole) de 5-fluoro-1-[(pyridin-4-yl)méthyl]-1*H*-indole-2-carboxylate d'éthyle, obtenu à la première étape de l'exemple 1. On porte à reflux pendant 3 h puis on agite le milieu réactionnel à température ambiante toute la nuit. On le verse sur de la glace et on extrait par deux fois avec 30 mL de dichlorométhane. Les phases organiques sont réunies, lavées avec 50 mL d'eau puis séchées sur sulfate de sodium, filtrées et concentrées sous pression réduite. On purifie le résidu par chromatographie préparative (éluant : dichlorométhane - acétone). On obtient 0,46 g d'un solide qui est séché sous pression réduite.
Point de fusion (base) : 249 - 250 °C
Le solide résultant est repris dans 30 mL de dichlorométhane et 0,26 mL d'une solution d'acide chlorhydrique 4N dans le dioxane est ajouté. La solution est concentrée sous pression réduite et le solide résultant recristallisé dans un mélange d'isopropanol et de méthanol. On obtient ainsi 0,33 g du produit attendu sous la forme d'un chlorhydrate.
Point de fusion (2HCl): 285 - 287 °C
R.M.N. $^1$H (DMSO $D_6$), $\delta$ (ppm): 2,8 (s, 3H), 3,87 (s, 3H) ; 6,1 (s, 2H) ; 7,16 (dxd, 1H); 7,51 (m, 4H) ; 7,75 (s, 1 H) ; 7,85 (d, 2H) ; 8,3 (s, 1H);8,75 (d, 2H).

**Exemple 5 (Composé N°11)**

*N*-(1-méthyl-1*H*-indol-5-yl)-5-fluoro-1-[(pyridin-3-yl)éthyl]-1*H*-indole-2-carboxamide

5.1. 5-fluoro-1-[(pyridin-3-yl)éthyl]-1*H*-indole-2-carboxylate d'éthyle

On ajoute, goutte à goutte, une solution de 0,365 g (1,44 mmole) de 1,1'-(azodicarbonyl)dipipéridine dans 10 mL de tétrahydrofurane sur une solution de 0,2 g (0,97 mmole) de 5-fluoro-1*H*-indole-2-carboxylate d'éthyle, de 0,178 g (1,45 mmole) de 2-(pyridin-3-yl)éthanol et de 0,36 mL (1,44 mmole) de tributylphosphine dans 30 mL de tétrahydrofurane. Le mélange réactionnel est agité une nuit à température ambiante puis concentré sous pression réduite et repris dans 50 mL de cyclohexane. La suspension est ensuite filtrée et le filtrat est chromatographié sur colonne de silice (éluant : dichloromethane - méthanol). On obtient 0,125 g de produit attendu.

5.2 *N*-(1-méthyl-1*H*-indol-5-yl)-5-fluoro-1-[(pyridin-3-yl)éthyl]-1*H*-indole-2-carboxamide (composé n°11)

On ajoute, à 0°C sous argon, à une solution de 0,7 g (0,478 mmole) de 5-amino-1-méthyl-1*H*-indole (I.T. Forbes, J.Med.Chem. 1993, 36 (8), 1104) dans 5 mL de toluène sec, 0,54 mL de triméthylaluminium (2M dans le toluène). Après 15 min, on ajoute 0,125 g (0,4 mmole) de 5-fluoro-1-[(pyridin-3-yl)éthyl]-1*H*-indole-2-carboxylate d'éthyle, obtenu à

l'étape 5.1. On porte à reflux pendant 5 h puis on agite le milieu réactionnel à température ambiante toute la nuit. On le verse sur 50 g de glace, 10 mL d'acide chlorhydrique 1 N et 30 mL d'acétate d'éthyle. La phase organique est séparée et on additionne 15 mL de soude 1 N à la phase aqueuse que l'on réextrait avec 30 mL d'acétate d'éthyle supplémentaires. Les phases organiques sont réunies, lavées avec une solution saturée en chlorure de sodium, séchées sur sulfate de sodium et concentrées sous pression réduite. Le résidu obtenu est repris dans 15 ml d'éther isopropylique et l'insoluble est filtré puis séché sous pression réduite. On isole ainsi 50 mg du produit attendu sous la forme d'un solide.
Point de fusion (base) : 188 - 189 °C
R.M.N. $^1H$ ($CDCl_3$), $\delta$ (ppm): 3,19 (t, 2H) ; 3,85 (s, 3H) ; 4,85 (t, 2H); 6,49 (d, 1H) ; 6,92 (s, 1 H) ; 7,1 (m, 3H); 7,29 (m, 4H) ; 7,49 (dxt, 1 H) ; 7,75 (m, 1 H) ; 7,9 (s, 1 H) ; 8,21 (d, 1 H); 8,4 (d, 1 H).

**Exemple 6 (Composé N°94, 95 et 96)**

*N*-(1,2-diméthyl-1*H*-benzimidazol-5-yl)-5-fluoro-1-[(3,6-diméthyl-pyrazin-2-yl)méthyl]-1*H*-indole-2-carboxamide (Composé n°94)
*N*-(1,2-diméthyl-1*H*-benzimidazol-5-yl)-5-fluoro-1-[(5,6-diméthyl-pyrazin-2-yl)méthyl]-1*H*-indole-2-carboxamide (Composé n° 95)
*N*-(1,2-diméthyl-1*H*-benzimidazol-5-yl)-5-fluoro-1-[(3,5-diméthyl-pyrazin-2-yl)méthyl]-1*H*-indole-2-carboxamide (Composé n° 96)

6.1. (Chlorométhyl)-diméthylpyrazine (mélange de 3 isomères)

On ajoute, en 1 h, 7,6 g (32,74 mmoles) d'acide trichloroisocyanurique sur une solution, portée à reflux, de 10 g de triméthylpyrazine (81,85 mmoles) dans 820 mL de dichloroéthane. Le mélange réactionnel est chauffé à reflux durant 6 h puis refroidit à 20°C, agité 12 h supplémentaires et filtré. Le filtrat est concentré sous pression réduite, repris dans 200 mL d'éther éthylique, filtré à nouveau puis concentré sous pression réduite. On récupère 9,6 de chlorure de méthyl-diméthyl-pyrazine, sous la forme d'un mélange que l'on engage tel quel dans l'étape suivante.

6.2. 5-fluoro-1-[(diméthyl-pyrazin-2-yl)méthyl]-1*H*-indole-2-carboxylate d'éthyle (mélange de composés $IV_{21}$)

On ajoute, goutte à goutte, une solution de 5 g (24,13 mmoles) de 5-fluoro-1*H*-indole-2-carboxylate d'éthyle sur une suspension de 1,45 g (36,2 mmoles) d'hydrure de sodium à 60% dans 200 mL de diméthylformamide, agitée à 0°C sous argon. Le mélange est agité 30 min à 0°C puis 1 h à 20°C. On ajoute ensuite, en plusieurs portions, 9.45 g (60.33 mmoles) du mélange de chlorure de méthyl-diméthyl-pyrazine obtenu à l'étape précédente. Le mélange est agité 2 h à 20°C puis on ajoute 200 mL d'eau et 200 mL d'acétate d'éthyle. La phase organique est séparée et la phase aqueuse réextraite avec 100 mL d'acétate d'éthyle. Les phases organiques sont réunies, lavées avec 2 fois 50 mL d'eau, 50 mL d'une solution aqueuse saturée en chlorure de sodium puis séchées sur sulfate de magnésium, filtrées et concentrées sous pression réduite. On purifie le résidu par chromatographie préparative (éluant : dichlorométhane - acétate d'éthyle).
On obtient 1,1 g d'un mélange des trois isomères attendus ($IV_{21}$), utilisé tel quel dans la suite de la synthèse.

6.3. *N*-(1,2-diméthyl-1*H*-benzimidazol-5-yl)-5-fluoro-1-[(3,6-diméthyl-pyrazin-2-yl)méthyl]-1*H*-indole-2-carboxamide (Composé n°94)

*N*-(1,2-diméthyl-1*H*-benzimidazol-5-yl)-5-fluoro-1-[(5,6-diméthyl-pyrazin-2-yl)méthyl]-1*H*-indole-2-carboxamide (Composé n° 95)
*N*-(1,2-diméthyl-1*H*-benzimidazol-5-yl)-5-fluoro-1-[(3,5-diméthyl-pyrazin-2-yl)méthyl]-1H-indole-2-carboxamide (Composé n° 96)

On ajoute sous argon, à une solution de 0,568 g (3,53 mmoles) de 5-amino-1,2-diméthyl-1*H*-benzimidazole (WO02059110) dans 20 mL de toluène sec, 2,57 mL de triméthylaluminium (2M dans le toluène). Après 15 min, on ajoute 1,05 g (3,21 mmoles) de 5-fluoro-1-[(diméthyl-pyrazin-2-yl)méthyl]-1*H*-indole-2-carboxylate d'éthyle, obtenu à l'étape précédente. On porte à reflux pendant 4 h puis on agite le milieu réactionnel à température ambiante toute la nuit. On le verse sur de la glace et on extrait par deux fois avec 100 mL d'acétate d'éthyle. Les phases organiques sont réunies, successivement lavées avec 50 mL d'eau et 50 mL d'une solution aqueuse saturée en chlorure de sodium puis

séchées sur sulfate de magnésium, filtrées et concentrées sous pression réduite. On purifie le résidu par chromatographie HPLC préparative (éluants : Ethanol - Heptane - triéthylamine).

On sépare :

- 0,59 g de l'isomère n°94 sous la forme d'un solide,
  Point de fusion : 269 - 275 °C
  R.M.N. $^1$H (DMSO $D_6$), δ (ppm): 2,31 (s, 3H) ; 2,46 (s, 3H) ; 2,6 (s, 3H) ; 3,69 (s, 3H) ; 5,95 (s, 2H) ; 7,02 (txd, 1) ; 7,4 (m, 5H) ; 7,86 (s, 1 H) ; 8 (s, 1 H)
- 0,139 g de l'isomère n° 95 sous la forme d'un solide,
  Point de fusion : 226 - 228 °C
  R.M.N. $^1$H (DMSO $D_6$), δ (ppm): 2,36 (s, 6H) ; 2,49 (s, 3H) ; 3,9 (s, 3H) ; 6,41 (s, 2H) ; 7,8 (txd, 1H); 8,06 (s, 1 H) ; 8,2 (m, 1 H) ; 8,29 (m, 2H) ; 8,4 (m, 1 H) ; 8,71 (s, 2H) ; 11,6 (s, 1 H)
- et 0,51 g de l'isomère n° 96 sous la forme d'un solide.
  Point de fusion : 266 - 269 °C
  R.M.N. $^1$H (DMSO $D_6$), δ (ppm): 2,18 (s, 3H) ; 2,49 (s, 3H) ; 2,6 (s, 3H) ; 3,71 (s, 3H) ; 6,41 (s, 2H) ; 7,1 (txd, 1 H) ; 7,4 (m, 5H) ; 7,85 (s, 1 H) ; 8,2 (s, 1 H) ; 10,3 (s, 1 H).

**Exemple 7 (Composé N°32)**

*N*-(1,2-diméthyl-1*H*-benzimidazol-5-yl)-5-fluoro-1-[(pyrazin-2-yl)méthyl]-1*H*-indole-2-carboxamide (Composé n°32)

7.1. 5-fluoro-1-[(pyrazin-2-yl)méthyl]-1*H*-indole-2-carboxylate d'éthyle (composé n° $IV_{14}$)

Selon une méthode analogue à celle décrite à l'exemple 6.2, on isole 1,55 g de 5-fluoro-1-[(pyrazin-2-yl)méthyl]-1*H*-indole-2-carboxylate d'éthyle (composé n° $IV_{14}$) à partir de 2 g (9,65 mmoles) de 5-fluoro-1*H*-indole-2-carboxylate d'éthyle
$[MH]^+$ = 300

7.2. *N*-(1,2-diméthyl-1*H*-benzimidazol-5-yl)-5-fluoro-1-[(pyrazin-2-yl)méthyl]-1*H*-indole-2-carboxamide (Composé n°32)

Selon une méthode analogue à celle décrite à l'exemple 6.3, on isole 0,2 g de *N*-(1,2-diméthyl-1*H*-benzimidazol-5-yl)-5-fluoro-1-[(pyrazin-2-yl)méthyl]-1*H*-indole-2-carboxamide (Composé n°32) à partir de 0,5 g de 5-fluoro-1-[(pyrazin-2-yl)méthyl]-1*H*-indole-2-carboxylate d'éthyle (composé n° $IV_{14}$).
Point de fusion : 239 - 240 °C
R.M.N. $^1$H (DMSO $D_6$), δ (ppm): 2,5 (s, 3H) ; 3,7 (s, 3H) ; 5,98 (s, 2H) ; 7,11 (txd, 1H); 7,49 (m, 5H) ; 7,9 (d, 1 H) ; 8,4 (s, 1 H) ; 8,5 (s, 2H) ; 10,39 (s, 1 H).

**Exemple 8 (Composé N°37)**

*N*-(1',2-diméthy)-1*H*-benzimidazol-5-yl)-5-fluoro-1-[(4-méthyl-pyridin-2-yl)méthyl]-1*H*-indole-2-carboxamide (Composé n°37)

8.1. 5-fluoro-1-[(4-méthyl-pyridin-2-yl)méthyl]-1*H*-indole-2-carboxylate d'éthyle (composé n° $IV_{17}$)

Selon une méthode analogue à celle décrite à l'exemple 6.2, on isole 1,2 g de 5-fluoro-1-[(4-méthyl-pyridin-2-yl)méthyl]-1*H*-indole-2-carboxylate d'éthyle (composé n° $IV_{17}$) à partir de 2 g (9,65 mmoles) de 5-fluoro-1*H*-indole-2-carboxylate d'éthyle
$[MH]^+$ = 313

8.2. *N*-(1,2-diméthyl-1*H*-benzimidazol-5-yl)-5-fluoro-1-[(4-méthyl-pyridin-2-yl)méthyl]-1*H*-indole-2-carboxamide (Composé n°37)

Selon une méthode analogue à celle décrite à l'exemple 6.3, on isole 0,48 g de *N*-(1,2-diméthyl-1*H*-benzimidazol-5-yl)-5-fluoro-1-[(4-méthyl-pyridin-2-yl)méthyl]-1*H*-indole-2-carboxamide (Composé n°37) à partir de 0,51 g de 5-fluoro-1-[(4-méthyl-pyridin-2-yl)méthyl]-1*H*-indole-2-carboxylate d'éthyle (composé n° $IV_{17}$)
Point de fusion : 213 - 215 °C
R.M.N. $^1$H (DMSO $D_6$), δ (ppm): 2,18 (s, 3H) ; 2,49 (s, 3H) ; 3,67 (s, 3H) ; 5,88 (s, 2H); 6,82 (s, 1 H) ; 7,08 (m, 2H) ; 7,4 (m, 5H) ; 7,9 (s, 1 H) ; 8,39 (s, 1H) ;10,4 (s, 1 H).

**<u>Exemple 9</u> (Composé N°67)**

*N*-(1-méthyl-1*H*-indol-5-yl)-5-fluoro-1-[(pyrimidin-4-yl)méthyl]-1*H*-indole-2-carboxamide (Composé n°67)

9.1. 5-fluoro-1-[(pyrimidin-4-yl)méthyl]-1*H*-indole-2-carboxylate d'éthyle (composé n° $IV_{35}$)

Selon une méthode analogue à celle décrite à l'exemple 6.2, on isole 2,6 g de 5-fluoro-1-[(pyrimidin-4-yl)méthyl]-1*H*-indole-2-carboxylate d'éthyle (composé n° $IV_{35}$) à partir de 3,3 g (15,93 mmoles) de 5-fluoro-1*H*-indole-2-carboxylate d'éthyle.
$[MH]^+$ = 300

9.2. *N*-(1-méthyl-1*H*-indol-5-yl)-5-fluoro-1-[(pyrimidin-4-yl)méthyl]-1*H*-indole-2-carboxamide (Composé n°67)

Selon une méthode analogue à celle décrite à l'exemple 6.3, on isole 0,41 g de *N*-(1-méthyl-1*H*-indol-5-yl)-5-fluoro-1-[(pyrimidin-4-yl)méthyl]-1*H*-indole-2-carboxamide
(Composé n°67) à partir de 0,5 g de 5-fluoro-1-[(pyrimidin-4-yl)méthyl]-1*H*-indole-2-carboxylate d'éthyle (composé n° $IV_{35}$).
Point de fusion : 199 - 200 °C
R.M.N. $^1H$ (DMSO $D_6$), $\delta$ (ppm): 2,5 (s, 3H) ; 3,69 (s, 3H) ; 5,93 (s, 2H) ; 6,91 (d, 1H); 7,08 (txd, 1 H) ; 7,42 (m, 5H) ; 7,85 (s, 1 H) ; 8,62 (d, 1 H) ; 9,03 (s, 1 H) ; 10,31 (s, 1 H).

**<u>Exemple 10</u> (Composé N°68)**

*N*-(1,2-diméthyl-1*H*-benzimidazol-5-yl)-5-fluoro-1-[(6-méthyl-pyrazin-2-yl)méthyl]-1*H*-indole-2-carboxamide (Composé n°68)

10.1. 5-fluoro-1-[(6-méthyl-pyrazin-2-yl)méthyl]-1*H*-indole-2-carboxylate d'éthyle (composé n° $IV_{22}$)

Selon une méthode analogue à celle décrite à l'exemple 6.2, on isole 0,32 g de 5-fluoro-1-[(6-méthyl-pyrazin-2-yl)méthyl]-1*H*-indole-2-carboxylate d'éthyle (composé n° $IV_{22}$) à partir de 3,3 g (15,93 mmoles) de 5-fluoro-1*H*-indole-2-carboxylate d'éthyle $[MH]^+$ = 314

10.2. *N*-(1,2-diméthyl-1*H*-benzimidazol-5-yl)-5-fluoro-1-[(6-méthyl-pyrazin-2-yl)méthyl]-1*H*-indole-2-carboxamide (Composé n°68)

Selon une méthode analogue à celle décrite à l'exemple 6.3, on isole 0,28 g de *N*-(1,2-diméthyl-1*H*-benzimidazol-5-yl)-5-fluoro-1-[(6-méthyl-pyrazin-2-yl)méthyl]-1*H*-indole-2-carboxamide (Composé n°68) à partir de 0,38 g dé 5-fluoro-1-[(6-méthyl-pyrazin-2-yl)méthyl]-1*H*-indole-2-carboxylate d'éthyle (composé n° $IV_{22}$).
Point de fusion : 244 - 249 °C
R.M.N. $^1H$ (DMSO $D_6$), $\delta$ (ppm): 2,39 (s, 3H) ; 2,49 (s, 3H) ; 3,69 (s, 3H) ; 5,91 (s, 2H); 7,08 (m, 1 H) ; 7,42 (m, 5H) ; 7,9 (s, 1 H) ; 7,99 (s, 1 H) ; 8,36 (s, 1 H).

**<u>Exemple 11</u> (Composé N°24)**

*N*-(1,2-diméthyl-1*H*-benzimidazol-5-yl)-5-trifluorométhyl-1-[(pyridin-4-yl)méthyl]-1H-indole-2-carboxamide (Composé n°24)

11.1. 5-trifluorométhyl-1-[(pyridin-4-yl)méthyl]-1*H*-indole-2-carboxylate d'éthyle (composé n° $IV_{27}$)

On ajoute, goutte à goutte, une solution de 1,47 g (5,83 mmoles) de 1,1'-(azodicarbonyl)dipipéridine dans 50 mL de toluène sur une solution de 1 g (3,89 mmoles) de 5-trifluorométhyl-1*H*-indole-2-carboxylate d'éthyle, de 0,63 g (5,83 mmoles) de 4-pyridylcarbinol et de 1,46 mL (5,83 mmoles) de tributylphosphine dans 50 mL de toluène. Le mélange réactionnel est agité une nuit à température ambiante puis concentré sous pression réduite et chromatographié sur colonne de silice (éluant : dichloromethane - méthanol). On obtient 1.05 g de produit attendu.
R.M.N. $^1H$ (DMSO $D_6$), $\delta$ (ppm): 1,27 (t, 3H) ; 4,29 (q, 2H) ; 5,95 (s, 2H) ; 6,93 (d, 2H); 7,58 (s, 1 H) ; 7,61 (dxd, 1 H) ; 7,80 (d, 1 H) ; 8,22 (s, 1 H) ; 8,45 (dxd, 2H).

11.2. *N*-(1,2-diméthyl-1*H*-benzimidazol-5-yl)-5-trifluorométhyl-1-[(pyridin-4-yl)méthyl]-1*H*-indole-2-carboxamide (Composé n°24)

Selon une méthode analogue à celle décrite à l'exemple 6.3, on isole 0,65 g de *N*-(1,2-diméthyl-1*H*-benzimidazol-5-yl)-5-trifluorométhyl-1-[(pyridin-4-yl)méthyl]-1*H*-indole-2-carboxamide (Composé n°24) à partir de 0,5 g de 5-trifluorométhyl-1-[(pyridin-4-yl)méthyl]-1*H*-indole-2-carboxylate d'éthyle (composé n° $IV_{27}$).
Point de fusion : 250 - 251 °C
R.M.N. $^1$H (DMSO $D_6$), $\delta$ (ppm): 2,49 (s, 3H) ; 3,69 (s, 3H) ; 5,95 (s, 2H) ; 6,98 (d, 1H); 7,49 (m, 4H) ; 7,69 (d, 1 H) ; 7,9 (s, 1 H) ; 7,99 (s, 1 H) ; 8,19 (s, 1 H) ; 8,42 (d, 2H).

**Exemple 12 (Composé N°91)**

*N*-(2-cyclopropyl-1-méthyl-1*H*-benzimidazol-5-yl)-5-fluoro-1-[(pyridin-4-yl)méthyl]-1H-indole-2-carboxamide (Composé n°91)

12.1. 2-cyclopropyl-5-nitro-1*H*-benzimidazole

Par analogie avec une méthode décrite (WO96/04270), une solution de 5 g (32,65 mmoles) de 4-nitro-1,2-phenylenediamine dans 77 mL (0,979 mole) d'acide cyclopropane carboxylique est agitée à reflux durant 14 h. Le mélange réactionnel est ensuite concentré sous pression réduite puis repris dans 150 mL d'acétate d'éthyle et 100 mL d'hydrogénocarbonate de sodium. La phase organique est lavée avec 100 mL d'eau puis 50 mL d'une solution saturée en chlorure de sodium, séchée sur sulfate de magnésium puis concentrée sous pression réduite. On obtient 6.5 g d'une huile marron qui sera utilisée telle quelle à l'étape suivante.

12.2. 2-cyclopropyl-1-méthyl-5-nitro-1*H*-benzimidazole et 2-cyclopropyl-1-méthyl-6-nitro-1*H*-benzimidazole

On ajoute, goutte à goutte, à une suspension agitée, sous argon à température ambiante, de 2,6 g d'hydrure de sodium (63,98 mmoles) dans 150 mL de tétrahydrofuranne, une solution de 6,5 g (32 mmoles) de 2-cyclopropyl-5-nitro-1*H*-benzimidazole, obtenu à l'étape précédente, dans 20 mL de tétrahydrofuranne. Le mélange réactionnel est agité 3 h à 20 °C, puis 2,2 mL (35,2 mmoles) d'iodure de méthyle sont additionnés. L'agitation est maintenue 48 h supplémentaires. Le mélange réactionnel est ensuite concentré sous pression réduite puis directement chromatographié sur colonne de silice (éluant : dichlorométhane - méthanol). On isole ainsi 4,5 g du mélange d'isomères attendu sous la forme d'un solide qui sera utilisé tel quel à l'étape suivante.

12.3. 2-cyclopropyl-1-méthyl-5-amino-1*H*-benzimidazole (composé n° $V_2$) et 2-cyclopropyl-1-méthyl-6-amino-1*H*-benzimidazole (composé n° $V_5$)

On ajoute à une suspension, agitée à 20°C, de 0,3 g de palladium sur charbon à 10% dans 50 mL d'éthanol, 15 g de formiate d'ammonium (0,236 mole). A ce premier réacteur est relié un second où est vigoureusement agitée à 20°C une suspension de 4,5 g (20,7 mmoles) du mélange de 2-cyclopropyl-1-méthyl-5-nitro-1*H*-benzimidazole et 2-cyclopropyl-1-méthyl-6-nitro-1*H*-benzimidazole obtenu à l'étape précédente et de 0,4 g de palladium sur charbon à 10% dans 130 mL d'éthanol.
Après 48 heures d'agitation à 20°C, 15 g (0,236 mole) de formiate d'ammonium, 0,3 g de palladium sur charbon à 10% et 50 mL d'éthanol sont ajoutés au mélange qui est agité 24 h supplémentaires puis filtré sur un tampon de célite et concentré sous pression réduite pour obtenir 4 g d'une huile que l'on purifie par HPLC.
On sépare :

- 1,1 g de produit $V_2$,
  $[MH]^+ = 188$
  R.M.N. $^1$H (DMSO $D_6$), $\delta$ (ppm): 1,05 (m, 4H) ; 2,25 (m, 1H); 3,81 (s, 3H) ; 6,61 (dxd, 1 H) ; 6,72 (s, 1 H) ; 7,26 (d, 1 H). - 0,92 g de produit $V_5$.
  $[MH]^+ = 188$
  R.M.N. $^1$H (DMSO $D_6$), $\delta$ (ppm): 1,02 (m, 4H) ; 2,2 (m, 1H); 3,71 (s, 3H) ; 6,5 (dxd, 1H); 6,61 (s, 1 H) ; 7,19 (d, 1 H).

12.4. *N*-(2-cyclopropyl-1-méthyl-1*H*-benzimidazol-5-yl)-5-fluoro-1-[(pyridin-4-yl)méthyl]-1*H*-indole-2-carboxamide (composé n°91)

On ajoute, goutte à goutte, 1,26 mL d'une solution 2N de triméthylaluminium dans le toluène, sur une solution

de 0,376 g (2,01 mmoles) de 2-cyclopropyl-1-méthyl-5-amino-1H-benzimidazole ($V_2$) dans 70 mL de toluène agitée à 0°C sous argon. On porte ensuite le mélange à 50°C pendant 15 min puis on refroidit et on ajoute en plusieurs portions 0,5 g (1,68 mmole) de 5-fluoro-1-[(pyridin-4-yl)méthyl]-1*H*-indole-2-carboxylate d'éthyle obtenu à l'étape 1.1. Le mélange est agité 14 h à reflux puis refroidi. On ajoute 15 mL d'eau puis 25 mL d'une solution 1 N d'acide chlorhydrique. Le pH de la solution résultante est amené à pH>8 par ajouts de soude concentrée. On filtre un solide que l'on reprend avec 200 mL d'acétate d'éthyle et 100 mL d'eau. La phase organique est séparée, séchée sur sulftate de magnésium, concentrée sous pression réduite puis chromatographiée (éluants : dichlorométhane - méthanol) pour conduire à 0,3 g du produit n°91 attendu.

PF = 260 - 261 °C

R.M.N. $^1$H (DMSO $D_6$), $\delta$ (ppm): 1 (m, 4H) ; 2,19 (m, 1H) ; 3,82 (s, 3H) ; 5,9 (s, 2H) ; 7,02 (d, 2H) ; 7,12 (txd, 1H) ; 7,47 (m, 5H) ; 7,82 (d, 1 H) ; 8,42 (d, 2H).

Le tableau 3 qui suit illustre les structures chimiques et les propriétés physiques de quelques composés de formule générale (I) selon l'invention. Dans ce tableau :

- la colonne "PF" renseigne les points de fusion des produits en degrés Celsius (°C) ;
- dans la colonne « Sel/base », « - » représente un composé sous forme de base libre, alors que « HCl » représente un composé sous forme de chlorhydrate et le rapport entre parenthèses est le rapport (acide:base) ;
- t-Bu représente un groupe *tertio*-butyle et Me un groupe méthyle.

Tableau 3

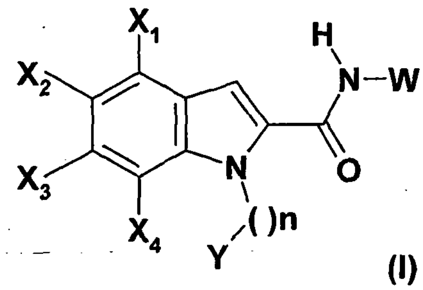

(I)

| N° | $X_1$, $X_2$, $X_3$, $X_4$ | n | Y | W | Sel/base | PF (°C) |
|---|---|---|---|---|---|---|
| 1 | H, F, H, H | 1 | pyridin-4-yle | 1-méthyl-benzimidazol-5-yle | HCl (2 :1) | 278 - 282 |
| 2 | H,F,H,H | 1 | pyridin-2-yle | 1-méthyl-benzimidazol-5-yle | HCl (2:1) | 255-260 |
| 3 | H, F, H, H | 1 | pyridin-4-yle | 1-méthyl-indol-5-yle | HCl (1:1) | 258-260 |
| 4 | H,F,H,H | 1 | pyridin-3-yle | 1-méthyl-benzimidazol-5-yle | HCl (2:1) | 268-270 |
| 5 | H, F, H, H | 1 | pyridin-3-yle | 1-méthyl-indol-5-yle | - | 214-215 |
| 6 | H, t-Bu, H, H | 1 | pyridin-4-yle | 1,2-diméthyl-benzimidazol-5-yle | HCl (2:1) | 295-300 |
| 7 | H,F,H,H | 1 | pyridin-4-yle | 1,2-diméthyl-benzimidazol-5-yle | HCl (2:1) | 285-287 |
| 8 | H, F, H, H | 1 | 2-méthyl-pyridin-3-yle | 1-méthyl-indol-5-yle | - | 249-250 |
| 9 | H, F, H, H | 1 | 6-méthyl-pyridin-2-yle | 1-méthyl-indol-5-yle | - | 205-206 |
| 10 | H, F, H, H | 1 | 2-morpholinyl-pvridin-3-yle | 1-méthyl-indol-5-yle | - | 232-234 |
| 11 | H, F, H, H | 2 | pyridin-3-yle | 1-méthyl-indol-5-yle | - | 188-189 |
| 12 | H, F, H, H | 1 | 1-méthyl-imidazol-2-yle | 1-méthyl-indol-5-yle | - | 177-179 |
| 13 | H, F, H, H | 1 | pyridin-2-yle | 1-méthyl-indol-5-yle | HCl (1:1) | 209-210 |
| 14 | H, F, H, H | 1 | 1-benzyl-imidazol-2-yle | 1-méthyl-indol-5-yle | - | 243-244 |

(suite)

| N° | $X_1$, $X_2$, $X_3$, $X_4$ | n | Y | W | Sel/base | PF (°C) |
|---|---|---|---|---|---|---|
| 15 | H, F, H, H | 2 | 4-méthyl-thiazol-5-yle | 1-méthyl-indol-5-yle | - | 205-206 |
| 16 | H, F, H, H | 1 | 1-méthyl-benzimidazol-2-yle | 1-méthyl-indol-5-yle | - | 260-262 |
| 17 | H, F, H, H | 1 | quinoléin-2-yle | 1-méthyl-indol-5-yle | - | 266-267 |
| 18 | H, F, H, H | 1 | pyidin-4-yle | indol-5-yle | - | 298-300 |
| 19 | H, H, $CF_3$, H | 1 | pyridin-4-yle | 1,2-diméthyl-benzimidazol-5-yle | - | 154-155 |
| 20 | H, F, H, H | 1 | quinoléin-2-yle | 1,2-diméthyl-benzimidazol-5-yle | -<br>HCl (1 :1) | 254-255<br>292-296 |
| 21 | H, F, H, H | 1 | 5-trifluorométhylfuran -2-yle | 1-méthyl-1,2,3,4-tétrahydroquinoléin-7-yle | | 237-238 |
| 22 | H, F, H, H | 1 | thiazol-2-yle | 1-méthyl-indol-5-yle | - | 236-238 |
| 23 | H, F, H, H | 1 | pyridin-4-yle | 2-méthyl-benzimidazol-5-yle | - | 300-305 |
| 24 | H, $CF_3$, H, H | 1 | pyridin-4-yle | 1,2-diméthyl-benzimidazol-5-yle | - | 250-251 |
| 25 | H, H, tBu, H | 1 | pyridin-4-yle | 1,2-diméthyl-benzimidazol-5-yle | - | 229-230 |
| 26 | H, F, H, H | 1 | benzothiazol-2-yle | 1-méthyl-indol-5-yle | - | 250-254 |
| 27 | H, F, H, H | 1 | benzothiazol-2-yle | 1,2-diméthyl-benzimidazol-5-yle | - | 270-271 |
| 28 | H, F, H, H | 1 | quinoxalin-2-yle | 1-méthyl-indol-5-yle | - | 212-214 |
| 29 | H, F, H, H | 2 | 2-methyl-pyridin-4-yle | 1,2-diméthyl-benzimidazol-5-yle | - | 249-250 |
| 30 | H, F, H, H | 1 | isoquinoléin-1-yle | 1,2-diméthyl-benzimidazol-5-yle | - | 263-268 |
| 31 | H, F, H, H | 1 | pyrazin-2-yle | 1-méthyl-indol-5-yle | - | 197-207 |
| 32 | H, F, H, H | 1 | pyrazin-2-yle | 1,2-diméthyl-benzimidazol-5-yle | - | 239 - 240 |
| 33 | H, F, H, H | 1 | pyridin-4-yle | 2-méthyl-indol-5-yle | - | 260-261 |
| 34 | H, F, H, H | 1 | 4-méthyl-pyridin-2-yle | 1-méthyl-indol-5-yle | - | 202-204 |
| 35 | H, F, H, H | 1 | quinoxalin-2-yle | 1,2-diméthyl-benzimidazol-5-yle | - | 243-247 |
| 36 | H, F, H, H | 1 | pyridin-4-yle | benzimidazol-5-yle | - | 255-257 |
| 37 | H, F, H, H , | 1 | 4-méthyl-pyridin-2-yle | 1,2-diméthyl-benzimidazol-5-yle | - | 213-215 |
| 38 | H, F, H, H | 1 | 2-méthyl-pyridin-4-yle | 1,2-diméthyl-benzimidazol-5-yle | - | 263 - 265 |
| 39 | H, H, $NMe_2$, H | 1 | pyridin-4-yle | 1,2-diméthyl-benzimidazol-5-yle | - | 255-256 |

(suite)

| N° | $X_1$, $X_2$, $X_3$, $X_4$ | n | Y | W | Sel/base | PF (°C) |
|---|---|---|---|---|---|---|
| 40 | H, F, H, H | 1 | pyridin-4-yle | 1-méthyl-2-trifluorométhyl-benzimidazol-5-yle | - | 224-226 |
| 41 | H, F, H, H | 1 | 3-méthyl-pyridin-2-yle | 1-méthyl-indol-5-yle | - | 198-199 |
| 42 | H, $CF_3$, H, H | 1 | pyridin-4-yle | indol-5-yle | - | 225-226 |
| 43 | H, $CF_3$, H, H | 1 | pyridin-4-yle | 1-méthyl-indol-5-yle | - | 385-386 |
| 44 | H, $CF_3$, H, H | 1 | pyridin-4-yle | 2-méthyl-benzimidazol-5-yle | - | 294-296 |
| 45 | H, $CF_3$, H, H | 1 | pyridin-4-yle | 2-méthyl-benzothiazol-5-yle | - | 233-234 |
| 46 | H, $CF_3$, H, H | 1 | pyridin-4-yle | quinoléin-7-yle | - | 395 - 397 |
| 47 | H, H, $NMe_2$, H | 1 | pyridin-4-yle | indol-5-yle | - | 277-278 |
| 48 | H, H, $NMe_2$, H | 1 | pyridin-4-yle | 1-méthyl-indol-5-yle | - | 226-227 |
| 49 | H, H, $NMe_2$, H | 1 | pyridin-4-yle | 2-méthyl-benzimidazol-5-yle | - | $[MH]^+$ 425 |
| 50 | H, H, $NMe_2$, H | 1 | pyridin-4-yle | 2-méthyl-benzothiazol-5-yle | - | 256-257 |
| 51 | H, H, $NMe_2$, H | 1 | pyridin-4-yle | quinoléin-7-yle | - | 266-267 |
| 52 | H, H, $CF_3$, H | 1 | pyridin-4-yle | indol-5-yle | - | 332-333 |
| 53 | H, H, $CF_3$, H | 1 | pyridin-4-yle | 1-méthyl-indol-5-yle | - | 230-231 |
| 54 | H, H, $CF_3$, H | 1 | pyridin-4-yle | 2-méthyl-benzimidazol-5-yle | - | 154-155 |
| 55 | H, H, $CF_3$, H | 1 | pyridin-4-yle | 2-méthyl-benzothiazol-5-yle | - | 252-253 |
| 56 | H, H, $CF_3$, H | 1 | pyridin-4-yle | quinoléin-7-yle | - | 237-238 |
| 57 | H, H, tBu, H | 1 | pyridin-4-yle | indol-5-yle | - | 255-256 |
| 58 | H, H, tBu, H | 1 | pyridin-4-yle | 1-méthyl-indol-5-yle | - | $[MH]^+$ 437 |
| 59 | H, H, tBu, H | 1 | pyridin-4-yle | 2-méthyl-benzimidazol-5-yle | - | 272-274 |
| 60 | H, H, tBu, H | 1 | pyridin-4-yle | 2-méthyl-benzothiazol-5-yle | - | 204-208 |
| 61 | H, H, tBu, H | 1 | pyridin-4-yle | quinoléin-7-yle | - | 280-282 |
| 62 | H, F, H, H | 1 | 3-méthyl-pyridin-2-yle | 1,2-diméthyl-benzimidazol-5-yle | - | 223-227 |
| 63 | H, F, H, H | 1 | pyridin-4-yle | 2-isopropyl-1-méthyl-benzimidazol-5-yle | - | 138-139 |
| 64 | H, F, H, H | 1 | 5-méthyl-pyridin-2-yle | 1-méthyl-indol-5-yle | - | 188-190 |
| 65 | H, F, H, H | 1 | 5-méthyl-pyridin-2-yle | 1,2-diméthyl-benzimidazol-5-yle | - | 222-223 |
| 66 | H, F, H, H | 1 | 6-méthyl-pyridazin-3-yle | 1-méthyl-indol-5-yle | - | 226-227 |
| 67 | H, F, H, H | 1 | pyrimidin-4-yle | 1-méthyl-indol-5-yle | - | 199 - 200 |

(suite)

| N° | $X_1$, $X_2$, $X_3$, $X_4$ | n | Y | W | Sel/base | PF (°C) |
|---|---|---|---|---|---|---|
| 68 | H, F, H, H | 1 | 6-méthyl-pyrazin-2-yle | 1,2-diméthyl-benzimidazol-5-yle | - | 244-249 |
| 69 | H, F, H, H | 2 | pyridin-3-yle | 1,2-diméthyl-benzimidazol-5-yle | - | 234-235 |
| 70 | H, tBu, H, H | 1 | pyridin-2-yle | indol-5-yle | - | 188-190 |
| 71 | H, tBu, H, H | 1 | pyridin-2-yle | 1-méthyl-indol-5-yle | - | 161-163 |
| 72 | H, tBu, H, H | 1 | pyridin-2-yle | 1-méthyl-benzimidazol-5-yle | - | 223-225 |
| 73 | H, tBu, H, H | 1 | pyridin-2-yle | 4-méthyl-3-oxo-2*H*-benzo[1,4]oxazin-6-yle | - | 136-138 |
| 74 | H, tBu, H, H | 1 | pyridin-2-yle | 1,2-diméthyl-benzimidazol-5-yle | - | 272-274 |
| 75 | H, tBu, H, H | 1 | pyridin-2-yle | 2-méthyl-benzimidazol-5-yle | - | 222-224 |
| 76 | H, tBu, H, H | 1 | pyridin-2-yle | 2-méthyl-benzothiazol-5-yle | - | 176-178 |
| 77 | H, tBu, H, H | 1 | pyridin-2-yle | quinoléin-7-yle | - | 232-234 |
| 78 | H, tBu, H, H | 1 | pyridin-2-yle | 1-méthyl-1,2,3,4-tétrahydroquinoléin-7-yle | - | 123-125 |
| 79 | H, tBu, H, H | 1 | pyridin-2-yle | 3-oxo-2*H*-benzo[1,4] oxazin-6-yle | - | $[MH]^+$ 455 |
| 80 | H, tBu, H, H | 1 | pyridin-3-yle | indol-5-yle | - | $[MH]^+$ 423 |
| 81 | H, tBu, H, H | 1 | pyridin-3-yle | 1-méthyl-indol-5-yle | - | 291-292 |
| 82 | H, tBu, H, H | 1 | pyridin-3-yle | 1-méthyl-benzimidazol-5-yle | - | 272-274 |
| 83 | H, tBu, H, H | 1 | pyridin-3-yle | 4-méthyl-3-oxo-2*H*-benzo[1,4]oxazin-6-yle | - | 125-127 |
| 84 | H, tBu, H, H | 1 | pyridin-3-yle | 1,2-diméthyl-benzimidazol-5-yle | - | 279-281 |
| 85 | H, tBu, H, H | 1 | pyridin-3-yle | 2-méthyl-benzimidazol-5-yle | - | 235-237 |
| 86 | H, tBu, H, H | 1 | pyridin-3-yle | 2-méthyl-benzothiazol-5-yle | - | 235-237 |
| 87 | H, tBu, H, H | 1 | pyridin-3-yle | quinoléin-7-yle | - | 242-244 |
| 88 | H, tBu, H, H | 1 | pyridin-3-yle | 1-méthyl-1,2,3,4-tétrahydroquinoléin-7-yle | - | 99 - 101 |
| 89 | H, tBu, H, H | 1 | pyridin-3-yle | 3-oxo-2*H*-benzo[1,4] oxazin-6-yle | - | 153-155 |
| 90 | H, F, H, H | 1 | pyridin-4-yle | 1-cyclopropylméthyl-2-méthyl-benzimidazol-6-yle | - | 282-284 |

(suite)

| N° | $X_1$, $X_2$, $X_3$, $X_4$ | n | Y | W | Sel/base | PF (°C) |
|---|---|---|---|---|---|---|
| 91 | H, F, H, H | 1 | pyridin-4-yle | 2-cyclopropyl-1-méthyl-benzimidazol-5-yle | - | 260-261 |
| 92 | H, F, H, H | 1 | 6-méthyl-pyridazin-3-yle | 1,2-diméthyl-benzimidazol-5-yle | - | 240-243 |
| 93 | H, F, H, H | 1 | pyrimidin-4-yle | 1,2-diméthyl-benzimidazol-5-yle | - | 242-244 |
| 94 | H, F, H, H | 1 | 3,6-diméthyl-pyrazin-2-yle | 1,2-diméthyl-benzimidazol-5-yle | - | 269-275 |
| 95 | H, F, H, H | 1 | 5,6-diméthyl-pyrazin-2-yle | 1,2-diméthyl-benzimidazol-5-yle | - | 226-228 |
| 96 | H, F, H, H | 1 | 3,5-diméthyl-pyrazin-2-yle | 1,2-diméthyl-benzimidazol-5-yle | - | 266-269 |
| 97 | H, F, H, H | 1 | pyridin-4-yle | 1-(cyclopropyl)méthyl-2-méthyl-benzimidazol-5-yle | - | 232-233 |
| 98 | H, tBu, H, H | 1 | pyridin-4-yle | 3-oxo-2*H*-benzo[1,4] oxazin-6-yle | - | 276-278 |
| 99 | H, tBu, H, H | 1 | pyridin-4-yle | 1-méthyl-1,2,3,4-tétrahydroquinoléin-7-yle | - | 292-293 |
| 100 | H, tBu, H, H | 1 | pyridin-4-yle | quinoléin-7-yle | - | 203-205 |
| 101 | H, tBu, H, H | 1 | pyridin-4-yle | indol-5-yle | - | 336-338 |
| 102 | H, tBu, H, H | 1 | pyridin-4-yle | 1-méthyl-indol-5-yle | - | 248-250 |
| 103 | H, tBu, H, H | 1 | pyridin-4-yle | 1-méthyl-benzimidazol-5-yle | - | 259-261 |
| 104 | H, tBu, H, H | 1 | pyridin-4-yle | 4-méthyl-3-oxo-2*H*-benzo[1,4]oxazin-6-yle | - | 218-220 |
| 105 | H, tBu, H, H | 1 | pyridin-4-yle | benzimidazol-5-yle | - | 292-293 |
| 106 | H, tBu, H, H | 1 | pyridin-4-yle | 2-méthyl-benzothiazol-5-yle | - | 255-257 |

Les composés de l'invention ont été soumis à des essais pharmacologiques *in vitro* et *in vivo* qui ont mis en évidence leur intérêt comme substances à activités thérapeutiques.

Les composés de l'invention présentent également des caractéristiques de solubilité dans l'eau qui favorise une bonne activité *in vivo.*

Test d'inhibition du courant induit par la capsaïcine sur les DRG de rat

- Culture primaire de cellules de ganglions de racine dorsale (DRG) de rat :
  Les neurones du DRG expriment naturellement le récepteur TRPV1.

Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 μg/ml gentamicine et 50 ng/ml de NGF, puis déposées sur des lamelles de verre recouvertes

de laminine (0.25 x 106 cellules par lamelle) qui sont ensuite placées dans des boîtes 12 puits Corning. Les cellules sont incubées à 37°C en atmosphère humidifiée contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

- Electrophysiologie :

Les chambres de mesure (volume 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont la sortie unique, constituée par un tube de polyéthylène (ouverture 500μm) est placée à moins de 3 mm de la cellule étudiée. La configuration "cellule entière" de la technique de patch-clamp à été utilisée. Les pipettes en verre borosilicaté (résistance 5-10 MOhms) sont approchées de la cellule grâce à un micromanipulateur piézoélectrique 3D ( Burleigh, PC1000). Les courants globaux (potentiel de membrane fixé à -60 mV) sont enregistrés avec un amplificateur Axopatch 1 D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.

L'application d'une solution de capsaïcine 300 nM, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à une concentration donnée (concentration de 10 nM ou de 0,1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.

Les pourcentages d'inhibition de la réponse capsaïcine (300 nM) sont compris entre 20% et 100% pour les composés les plus actifs de l'invention testés à la concentration de 10 nM à 0,1 nM (voir l'exemple du tableau 4).

Les composés de l'invention sont donc des antagonistes efficaces in vitro des récepteurs de type TRPV1.

Tableau 4

| N°composé | % inhibition en patch DRG |
|---|---|
| 6 | 80% (3 nM) |

Test d'irritation cornéenne souris

Le caractère irritant de la capsaïcine est aisément apprécié au niveau de la cornée puisque cet organe est un des plus innervés par les fibres C. Dans ce contexte, d'après des expériences préliminaires, l'application d'une très faible quantité de capsaïcine (2 μl à une concentration de 160 μM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance. On peut en conclure que la durée d'action de la capsaïcine à ces doses est inférieure à 2 minutes.

Résumé de la méthodologie :

Le principe de la série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans du Tween 80 à 10% dans le sérum physiologique. Il apparaît, à partir d'études contrôles que dans ces conditions, le solvant n'a aucun effet.

En pratique, le produit à tester est administré par voie orale, et, avec un délai (temps de prétraitement : t) qui dépend des données de pharmacocinétique, l'animal reçoit l'instillation oculaire de 2 μl d'une solution de capsaïcine à 160 μM préparée comme indiqué ci-dessus. Au cours d'une observation de 2 minutes suivant l'instillation, le nombre de frottements de l'oeil instillé par la patte antérieure ipsilatéral est répertorié. Pour un animal donné, le pourcentage de protection est calculé comme suit :

P= 100 – ((nombre de grattages observés / nombre moyen de grattages du groupe traité par le solvant) x 100)

Ce pourcentage de protection est moyenné pour chaque groupe d'animaux (n = nombre d'animaux testés avec le composé de l'invention).

Les pourcentages de protection évalués, dans ce modèle, pour les composés de l'invention les plus actifs, utilisés à la dose de 1 mg/kg *(po),* sont compris entre 20% et 100% (voir quelques exemples dans le tableau 5) :

Tableau 5

| n°composé | % P - (t) à 1 mg/kg *(po)* |
|---|---|
| 6 | 24 % - (1h) |
| 3 | 44 % - (1h) |

Les résultats de ces essais montrent que les composés les plus actifs de l'invention bloquent les effets induits par la stimulation des récepteurs TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formule (I), ou un sel pharmaceutiquement acceptable, ou encore un hydrate ou un solvate dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.

Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.

On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.

De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour la préparation d'un médicament destiné à traiter la dépression.

Les composés de l'invention peuvent également être utilisés pour la préparation d'un médicament destiné à traiter le diabète.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

## Revendications

**1.** Composé répondant à la formule (I)

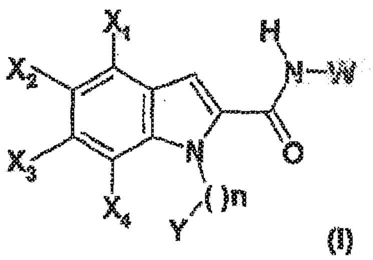

(I)

dans laquelle
$X_1$, $X_2$, $X_3$, $X_4$ représentent, indépendamment l'un de l'autre, un atome d'hydrogène ou d'halogène, ou un groupe $C_1$-$C_8$-alkyle, $C_1$-$C_8$-fluoroalkyle ou $NR_1R_2$,

W représente un groupe bicyclique fusionné de formule :

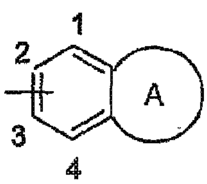

lié à l'atome d'azote par les positions 1,2,3 ou 4 ;

A représente un hétérocycle de 5 à 7 chaînons comprenant de un à trois hétéroatomes choisi parmi O, S ou N; le ou les atomes de carbones de A étant éventuellement substitués par un ou plusieurs groupes choisis parmi un atome d'hydrogène ou un groupe $C_1$-$C_8$-alkyle, $C_3$-$C_7$-cycloalkyle, $C_3$-$C_7$-cycloalkyle-$C_1$-$C_3$-alkylène, $C_1$-$C_8$-fluoroalkyle, aryle, aryle-$C_1$-$C_6$-alkylène, oxo ou thio;
le ou les atomes d'azote de A étant éventuellement substitués par $R_6$ lorsque l'azote est adjacent à un atome de carbone substitué par un groupe oxo, ou par $R_7$ dans les autres cas;
n est égal à 1,2 ou 3 ;
Y représente un hétéroaryle, choisi parmi les groupes pyridinyle, pyrimidinyle, pyrazinyle, pyridazinyle, imidazolyle, benzimidazolyle, benzothiazolyle, thiazolyle, furanyle, quinoléinyle, isoquinoléinyle, quinoxalinyle, l'hétéroaryle étant éventuellement substitué par un ou plusieurs groupes choisis parmi un groupe $C_1$-$C_6$-alkyle, $C_1$-$C_8$-fluoroalkyle, aryle-$C_1$-$C_6$-alkylène ou $NR_1R_2$ ; $R_1$ et $R_2$ formant ensemble, avec l'atome d'azote qui les porte, un groupe morpholinyle ;
$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-Cycloalkyle-$C_1$-$C_3$-alkylène, aryle-$C_1$-$C_6$-alkylène ou aryle ; ou $R_1$ et $R_2$ formant ensemble, avec l'atome d'azote qui les porte, un groupe azétidinyle, pyrrolidinyle, pipéridinyle, azépinyle, morpholinyle, thiomorpholinyle, pipérazinyle, homopipérazinyle, ce groupe étant éventuellement substitué par un groupe $C_1$-$C_8$-alkyle, $C_3$-$C_7$-cycloalkyle, $C_3$-$C_7$-cycloalkyle-$C_1$-$C_3$-alkylène, aryle-$C_1$-$C_8$-alkylène ou aryle ;
$R_6$ représente un atome d'hydrogéne ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_8$-alkylène ou aryle ;
$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ; le ou les atomes de soufre de l'hétérocycle A ou de l'hétéroaryle Y pouvant être sous forme oxydée;
le ou les atomes d'azote de l'hétérocycle A ou de l'hétéroaryle Y pouvant être sous forme oxydée ;
à l'état de base ou de sel d'addition à un acide, ainsi qu'à l'état d'hydrate ou de solvate.

2. Composé de formule (I) selon la revendication 1 **caractérisé en ce que**

W représente un groupe bicyclique fusionné de formule :

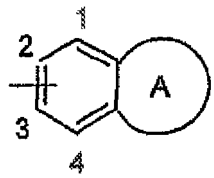

lié à l'atome d'azote par les positions 1, 2, 3 ou 4 ;
et W est choisi parmi les groupes indolinyle, isoindolyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indolyle, indazolyle, benzothiazolyle, isobenzothiazolyle, dihydroisabonzothiazolyle, bonzotriazolyle, quinoléinyle, dihydroquinoléinyle, tétrahydroquinoléinyle, isoquinoléinyle, dihydroisoquinoléinyle, tetrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ;
le ou les atomes de carbone et/ou d'azote dudit groupe W étant éventuellement substitués comme défini dans la formule générale (I) selon la revendication 1 ;
à l'état de base ou de sel d'addition à un acide, ainsi qu'à l'état d'hydrate ou de solvate.

3. Composé de formule (I) selon l'une quelconque des revendications 1 à 2, **caractérisé en ce que** W représente un groupe bicyclique fusionné de formule :

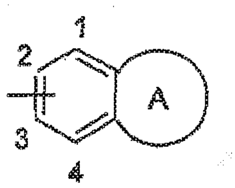

lié à l'atome d'azote par les positions 2 ou 3 ; et W est choisi parmi les groupes benzimidazolyle, indolyle, benzothiazolyle, quinoléinyle, tétrahydroquinoléinyle et benzoxazinyle ; et/ou le ou les atomes de carbones de A étant éventuellement substitués par un ou plusieurs groupes $C_1$-$C_8$-alkyle, $C_1$-$C_8$-fluoroalkyle, $C_3$-$C_7$-cycloalkyle ou oxo ; et/ou le ou les atomes d'azote de A étant éventuellement substitués par $R_6$ lorsque l'azote est adjacent à un atome de carbone substitué par un groupe oxo, $R_6$ représentant un atome d'hydrogène ou un groupe $C_1$-$C_8$-alkyle ;ou par $R_7$ dans les autres cas, $R_7$ représentant un groupe $C_1$-$C_6$-alkyle ou un groupe $C_3$-$C_7$-cycloalkyle-$C_1$-$C_3$-alkylène ;
à l'état de base ou de sel d'addition à un acide, ainsi qu'à l'état d'hydrate ou de solvate.

**4.** Composé de formule (I) selon l'une quelconque des revendications 1 à 3, **caractérisé en ce que** n est égal à 1 ou 2 ; à l'état de base ou de sel d'addition à un acide, ainsi qu'à l'état d'hydrate ou de solvate.

**5.** Composé de formule (I) selon la revendication 1, **caractérisé en ce que**
$R_1$ et $R_2$ étant tels que définis dans la formule générale (I) selon la revendication 1;
W représente un groupe bicyclique fusionné de formule :

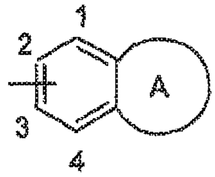

lié à l'atome d'azote par les positions 2 ou 3 ; et W est choisi parmi les groupes benzimidazolyte, indolyle, benzothiazolyle, quinoléinyle, tétrahydroquinoléinyle, benzoxazinyle ;
le ou les atomes de carbones de A étant éventuellement substitués par un ou plusieurs groupes $C_1$-$C_6$-alkyle, $C_1$-$C_6$-fluoroalkyle, $C_3$-$C_7$-cycloalkyle ou oxo ; et/ou le ou les atomes d'azote de A étant éventuellement substitués par $R_6$ lorsque l'azote est adjacent à un atome de carbone substitué par un groupe oxo, $R_8$ représentant un atome d'hydrogène ou un groupe $C_1$-$C_8$-alkyle ; ou par $R_7$ dans les autres cas, $R_7$ représentant un groupe $C_1$-$C_8$-alkyle ou un groupe $C_3$-$C_7$-cycloalkyle-$C_1$-$C_3$-alkylène ;
et/ou
n est égal à 1 ou 2 ; et/ou

à l'état de base ou de sel d'addition à un acide, ainsi qu'à l'état d'hydrate ou de solvate.

**6.** Procédé de préparation d'un composé de formule (I) selon la revendication 1, **caractérisé en ce que** l'on fait réagir un composé de formule générale (IV)

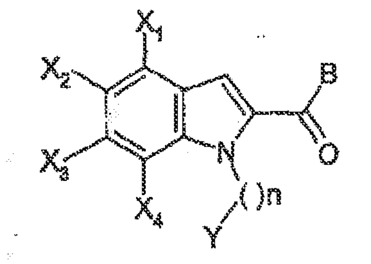

(IV)

dans laquelle $X_1$, $X_2$, $X_3$, $X_4$, Y et n sont tels que définis dans la formule générale (I) selon la revendication 1 et B

représente un groupe $C_1$-$C_6$-alcoxyle,
avec un amidure du composé de formule générale (V)

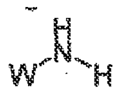

(V)

dans laquelle W est tel que défini dans la formule générale (I) selon la revendication 1, au reflux d'un solvant, l'amidure du composé de formule générale (V) étant préparé par action préalable du triméthylaluminium sur les amines de formule générale (V).

**7.** Composé de formule (IV) choisi parmi les composés suivants :

- 5-fluoro-1-[(thiazol-2-yl)méthyl]-1*H*-indole-2-carboxylate d'éthyle
- 5-fluoro-1-[(pyridin-3-yl)éthyl]-1*H*-indole-2-carboxylate d'éthyle
- 5-fluoro-1-[(pyridin-3-yl)méthyl]-1*H*-indole-2-carboxylate d'éthyle
- 5-fluoro-1-[(pyridin-4-yl)méthyl]-1*H*-indole-2-carboxylate d'éthyle
- 5-fluoro-1-[(pyridin-2-yl)méthyl]-1*H*-indole-2-carboxylate d'éthyle
- 5-fluoro-1[(2-méthyl-pyridin-3-yl)méthyl]-1*H*-indole-2-carboxylate d'éthyle
- 5-fluoro-1-[(1-N-benzyl-imidazol-2-yl)méthyl]-1*H*-indole-2-carboxylate d'éthyle
- 5-fluoro-1-[(2-pyrollidino-pyridin-3-yl)méthyl]-1*H*-indole-2-carboxylate d'éthyle
- 5-fluoro-1-[(benzothiazol-2-yl)méthyl]-1*H*-indole-2-carboxylate d'éthyle
- 5-fluoro-1-[(1-méthyl-benzimidazol-2-yl)méthyl]-1*H*-indole-2-carboxylate d'éthyle
- 5-fluoro-1-[(4-méthyl-thiazol-5-yl)éthyl]-1*H*-indole-2-carboxylate d'éthyle
- 5-fluoro-1-[(quinoléin-2-yl)-méthyl]-1*H*-indole-2-Carboxylate d'éthyle
- 5-fluoro-1-[(quinoxalin-2-yl)méthyl]-1*H*-indole-2-carboxylate d'éthyle
- 5-fluoro-1-[(pyrazin-2-yl)méthyl]-1*H*-indole-2-carboxylate d'éthyle
- 5-fluoro-1-[(3-méthyl-pyridin-2-yl)méthyl]-1*H*-indole-2-carboxylate d'éthyle
- 5-fluoro-1-[(isoquinoléin-1-yl)méthyl]-1*H*-indole-2-carboxylate d'éthyle
- 5-fluoro-1-[(4-méthyl-pyridin-2-yl)méthyl]-1*H*-indole-2-carboxylate d'éthyle
- 5-fluoro-1-[(2-méthyl-pyridin-2-yl)méthyl]-1*H*-indole-2-carboxylate d'éthyle 5-fluoro-1-[(2-méthyl-pyridin-2-yl) méthyl]-1*H*-indole-2-carboxylate d'éthyle
- 5-fluoro-1-[(5-méthyl-pyridin-2-yl)méthyl]-1*H*-indole-2-carboxylate d'éthyle
- 5-fluoro-1-[(diméthyl-pyrazin-2-yl)méthyl]-1*H*-indole-2-carboxylate d'éthyle
- 5-fluoro-1-[(6-méthyl-pyrazin-2-yl)méthyl]-1*H*-indole-2-carboxylate d'éthyle
- 5-fluoro-1-[(6-méthyl-pyridazin-3-yl)méthyl]-1*H*-indole-2-carboxylate d'éthyle
- 5-fluoro-1-[(6-méthyl-pyridin-2-yl)méthyl]-1*H*-indole-2-carboxylate d'éthyle
- 5-fluoro-1-[(2-phényl-pyridin-4-yl)méthyl]-1*H*-indole-2-carboxylate d'éthyle
- 6-terbutyl-1-[(pyridin-4-yl)méthyl]-1*H*-indole-2-carboxylate de méthyle
- 5-trifluorométhyl-1-[(pyridin-4-yl)méthyl]-1*H*-indole-2-carboxylate d'éthyle
- 6-N-diméthylamino-1-[(pyridin-4-yl)méthyl]-1*H*-indole-2-carboxylate d'éthyle
- 6- trifluorométhyl -1-[(pyridin-4-yl)méthyl]-1*H*-indole-2-carboxylate de méthyle
- 5-terbutyl-1-[(pyridin-4-yl)méthyl]-1*H*-indole-2-carboxylate d'éthyle
- 5-terbutyl-1-[(pyridin-2-yl)méthyl]-1H-indole-2-carboxylate d'éthyle
- 5-terbutyl-1-[(pyridin-3-yl)méthyl]-1*H*-indole-2-carboxylate d'éthyle
- 5-terbutyl-1-[(2-méthyl-pyridin-3-yl)méthyl]-1*H*-indole-2-carboxylate d'éthyle
- 5-terbutyl-1[(6-méthyl-pyridin-2-yl)méthyl]-1*H*-indole-2-carboxylate d'éthyle
- 5-fluoro-1-[(pyrimidin-4-yl)méthyl]-1*H*-indole-2-carboxylate d'éthyle à l'état de base ou de sel d'addition à un acide, ainsi qu'à l'état d'hydrate ou de solvate.

**8.** Composé de formule (V) choisi parmi les composés suivants:

- 5-amino-1-(cyclopropyl)méthyl-2-méthyl-1*H*-benzimidazole
- 5-amimo-2-cyclopropyl-1-méthyl-1*H*-benzimidazole
- 5-amino-2-isopropyl-1-méthyl-1*H*-benzimidazole
- 6-amine-1-(cyclopropyl)méthyl-2-méthyl-1*H*-benzimidazole

- 6-amino-2-cyclopropyl-1-méthyl-1*H*-benzimidazole
- 6-amino-2-isopropyl-1-méthyl-1*H*-benzimidazole

à l'état de base ou de sel d'addition à un acide, ainsi qu'à l'état d'hydrate ou de solvate.

**9.** Médicament, **caractérisé en ce qu'**il comprend un composé de formule (I), selon l'une quelconque des revendications 1 à 5, ou un sel pharmaceutiquement acceptable, ou encore un hydrate ou un solvate du composé de formule (I).

**10.** Composition pharmaceutique, **caractérisée en ce qu'**elle comprend un composé de formule (I), selon l'une quelconque des revendications 1 à 5, ou un sel pharmaceutiquement acceptable, un hydrate ou un solvate de ce composé, ainsi qu'au moins un excipient pharmaceutiquement acceptable.

**11.** Utilisation d'un composé de formule (I), selon l'une quelconque des revendications 1 à 5, pour préparation d'un médicament destiné à prévenir ou à traiter les pathologies dans lesquelles les récepteurs de type TRPV1 sont impliqués.

**12.** Utilisation d'un composé de formule (I), selon la revendication 11, pour la préparation d'un médicament destiné à prévenir ou à traiter la douleur, l'inflammation, les désordres urologiques, les désordres gynécologiques, les désordres gastrointestinaux, des désordres respiratoires, le psoriasis, le pruritis, les irritations dermiques, des yeux ou des muqueuses, l'herpès, le zona, ou à traiter la dépression ou le diabète.

**Patentansprüche**

**1.** Verbindung der Formel (I)

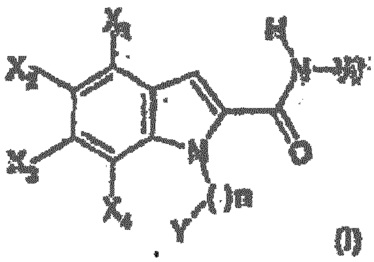

(I)

worin
$X_1$, $X_2$, $X_3$ und $X_4$ unabhängig voneinander für ein Wasserstoff- oder Halogenatom oder eine $C_1$-$C_6$-Alkyl-, $C_1$-$C_6$-Fluoralkyl- oder $NR_1R_2$-Gruppe stehen; W für eine anellierte bicyclische Gruppe der Formel:

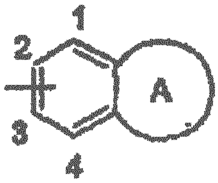

steht, die über die Positionen 1, 2, 3 oder 4 an das Stickstoffatom gebunden ist;
A für einen 5- bis 7-gliedrigen Heterocyclus mit einem bis drei aus O, S oder N ausgewählten Heteroatomen steht; wobei das Kohlenstoffatom bzw. die Kohlenstoffatome von A gegebenenfalls durch eine oder mehrere Gruppen substituiert ist bzw. sind, die unter einem Wasserstoffatom oder einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-, Aryl-$C_1$-$C_5$-alkylen-, Oxo- oder Thiogruppe ausgewählt sind;
wobei das Stickstoffatom bzw. die Stickstoffatome von A dann, wenn der Stickstoff neben einem durch eine Oxogruppe substituierten Kohlenstoffatom steht, gegebenenfalls durch $R_6$ oder in den anderen Fällen durch $R_7$ substituiert ist bzw. sind;
n gleich 1, 2 oder 3 ist;
Y für ein Heteroaryl steht, das aus Pyridinyl-, Pyrimidinyl-, Pyrazinyl-, Pyridazinyl-, Imidazolyl-, Benzimidazolyl-,

enzothiazolyl-, Thiazolyl-, Furanyl-, Chinolinyl-, Isochinolinyl- und Chinoxalinylgruppen ausgewählt ist, wobei das Heteroaryl gegebenenfalls durch eine oder mehrere Gruppen substituiert ist, die aus einer $C_1$-$C_6$-Alkyl-, $C_1$-$C_6$-Fluoralkyl-, Aryl-$C_1$-$C_6$-alkylen- oder $NR_1R_2$-Gruppe ausgewählt sind; wobei $R_1$ und $R_2$ gemeinsam mit dem Stickstoffatom, an das sie gebunden sind, eine Morpholinylgruppe bilden;
$R_1$ und $R_2$ unabhängig voneinander für ein Wasserstoffatom oder eine $C_1$-$C_6$-Alkylgruppe stehen;
$R_6$ für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-$C_1$-$C_6$-alkylen- oder Arylgruppe steht;
$R_7$ für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-$C_1$-$C_6$-alkylen-, $C_1$-$C_6$-Alkyl-C (O) -, $C_3$-$C_7$-Cycloalkyl-$C_1$-$C_3$-alkylen-C(O) -, $C_1$-$C_6$-Fluoralkyl-C(O)-, $C_3$-$C_7$-Cycloalkyl-C(O)-, Aryl-C(O)-, Aryl-$C_1$-$C_6$-alkylen-C(O)-, $C_1$-$C_6$-Alkyl-$S(O)_2$-, $C_1$-$C_6$-Fluoralkyl-S$(O)_2$-, $C_3$-$C_7$-Cycloalkyl-$S(O)_2$-, $C_3$-$C_7$-Cycloalkyl-$C_1$-$C_3$-alkylen-$S(O)_2$-, Aryl-$S(O)_2$-, Aryl-$C_1$-$C_6$-alkylen-$S(O)_2$- oder Aryl-gruppe steht;
wobei das Schwefelatom bzw. die Schwefelatome des Heterocyclus A oder des Heteroaryls Y in oxidierter Form vorliegen kann bzw. können;
wobei das Stickstoffatom bzw. die Stickstoffatome des Heterocyclus A oder des Heteroaryls Y in oxidierter Form vorliegen kann bzw. können;
in Basen- oder Säureadditionssalzform sowie in Hydrat- oder Solvatform.

2. Verbindung der Formel (I) nach Anspruch 1, **dadurch gekennzeichnet, dass**
W für eine anellierte bicyclische Gruppe der Formel:

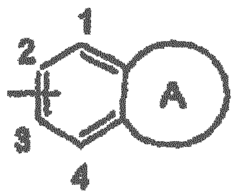

steht, die über die Positionen 1, 2, 3 oder 4 an das Stickstoffatom gebunden ist;
und W aus Indolinyl-, Isoindolyl, Isoindolinyl-, Benzofuranyl-, Dihydrobenzofuranyl-, Benzothiophenyl-, Dihydrobenzothiophenyl-, Benzoxazolyl-, Dihydrobenzoxazolinyl-, Isobenzofuranyl-, Dihydroisobenzofuranyl-, Benzimidazolyl-, Dihydrobenzimidazolyl-, Indolyl-, Indazolyl-, Benzothiazolyl-, Isobenzothiazolyl-, Dihydroisobenzothiazolyl-, Benzotriazolyl-, Chinolinyl-, Dihydrochinolinyl-, Tetrahydrochinolinyl-, Isochinolinyl-, Dihydroisochinolinyl-, Tetrahydroisochinolinyl-, Benzoxazinyl-, Dihydrobenzoxazinyl-, Benzothiazinyl-, Dihydrobenzothiazinyl-, Cinnolinyl-, Chinazolinyl-, Dihydrochinazolinyl-, Tetrahydrochinazolinyl-, Chinoxalinyl-, Dihydrochinoxalinyl-, Tetrahydrochinoxalinyl-, Phthalazinyl-, Dihydrophthalazinyl-, Tetrahydrophthalazinyl-, Tetrahydrobenz[*b*]azepinyl-, Tetrahydrobenz[c]azepinyl-, Tetrahydrobenz[*d*]azepinyl-, Tetrahydrobenzo[*b*]-[1,4]diazepinyl-, Tetrahydrobenzo[e][1,4]-diazepinyl-, Tetrahydrobenzo[*b*][1,4]oxazepinyl- oder Tetrahydrobenzo[*b*][1,4]thiazepinylgruppen ausgewählt ist; wobei das bzw. die Kohlenstoff- und/oder Stickstoffatom(e) der Gruppe A gegebenenfalls wie in der allgemeinen Formel (I) gemäß Anspruch 1 definiert substituiert ist bzw. sind;
in Basen- oder Säureadditionssalzform sowie in Hydrat- oder Solvatform.

3. Verbindung der Formel (I) nach Anspruch 1 oder 2, **dadurch gekennzeichnet, dass**
W für eine anellierte bicyclische Gruppe der Formel:

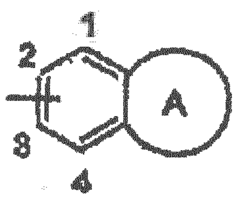

steht, die über die Positionen 2 oder 3 an das Stickstoffatom gebunden ist;
und W aus Benzimidazolyl-, Indolyl-, Benzothiazolyl-, Chinolinyl-, Tetrahydrochinolinyl- und Benzoxazinylgruppen ausgewählt ist; und/oder wobei das Kohlenstoffatom bzw. die Kohlenstoff-atome von A gegebenenfalls durch eine oder mehrere $C_1$-$C_6$-Alkyl-, $C_1$-$C_6$-Fluoralkyl-, $C_3$-$C_7$-Cycloalkyl- oder Oxogruppen substituiert ist bzw. sind; und/oder

wobei das Stickstoffatom bzw. die Stickstoffatome von A dann, wenn der Stickstoff neben einem durch eine Oxogruppe substituierten Kohlenstoffatom steht, gegebenenfalls durch $R_6$ oder in den anderen Fällen durch $R_7$ substituiert ist bzw. sind, wobei $R_6$ für ein Wasserstoffatom oder eine $C_1$-$C_6$-Alkylgruppe steht und $R_7$ für eine $C_1$-$C_6$-Alkyl- oder $C_3$-$C_7$-Cycloalkylen-$C_1$-$C_3$-alkylengruppe steht;
in Basen- oder Säureadditionssalzform sowie in Hydrat- oder Solvatform.

**4.** Verbindung der Formel (I) nach einem der Ansprüche 1 bis 3, **dadurch gekennzeichnet, dass** n gleich 1 oder 2 ist; in Basen- oder Säureadditionssalzform sowie in Hydrat- oder Solvatform.

**5.** Verbindung der Formel (I) nach Anspruch 1, **dadurch gekennzeichnet, dass**
$X_1$, $X_2$, $X_3$ und $X_4$ unabhängig voneinander für ein Wasserstoff- oder Halogenatom oder eine $C_1$-$C_6$-Alkyl-, $C_1$-$C_6$-Fluoralkyl- oder $NR_1R_2$-Gruppe stehen, wobei $R_1$ und $R_2$ die in der allgemeinen Formel (I) gemäß Anspruch 1 angegebene Bedeutung besitzen;
W für eine anellierte bicyclische Gruppe der Formel:

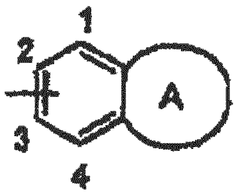

steht, die über die Positionen 2 oder 3 an das Stickstoffatom gebunden ist; und W aus Benzimidazolyl-, Indoxyl-, Benzothiazolyl-, Chinolinyl-, Tetrahydrochinolinyl- und Benzoxazinylgruppen ausgewählt ist;
wobei das Kohlenstoffatom bzw. die Kohlenstoff-atome von A gegebenenfalls durch eine oder mehrere $C_1$-$C_6$-Alkyl-, $C_1$-$C_6$-Fluoralkyl-, $C_3$-$C_7$-Cycloalkyl- oder Oxogruppen substituiert ist bzw. sind;
wobei das Stickstoffatom bzw. die Stickstoffatome von A dann, wenn der Stickstoff neben einem durch eine Oxogruppe substituierten Kohlenstoffatom steht, gegebenenfalls durch $R_6$ oder in den anderen Fällen durch $R_7$ substituiert ist bzw. sind, wobei $R_6$ für ein Wasserstoffatom oder eine $C_1$-$C_6$-Alkylgruppe steht und $R_7$ für eine $C_1$-$C_6$-Alkyl- oder $C_3$-$C_7$-Cycloalkylen-$C_1$-$C_3$-alkylengruppe steht; und/oder
n gleich 1 oder 2 ist; und/oder
Y für ein Heteroaryl steht, das aus Pyridinyl-, Pyrimidinyl-, Pyrazinyl-, Pyridazinyl-, Imidazolyl-, Benzimidazolyl-, Benzothiazolyl-, Thiazolyl-, Furanyl-, Chinolinyl-, Isochinolinyl- und Chinoxalinylgruppen ausgewählt ist, wobei das Heteroaryl gegebenenfalls durch eine oder mehrere Gruppen substituiert ist, die aus einer $C_1$-$C_6$-Alkyl-, $C_1$-$C_6$-Fluoralkyl-, Aryl-$C_1$-$C_6$-alkylen- oder $NR_1R_2$-Gruppe ausgewählt sind; wobei $R_1$ und $R_2$ gemeinsam mit dem Stickstoffatom, an das sie gebunden sind, eine Morpholinylgruppe bilden;
in Basen- oder Säureadditionssalzform sowie in Hydrat- oder Solvatform.

**6.** Verfahren zur Herstellung einer Verbindung der Formel (I) nach Anspruch 1, **dadurch gekennzeichnet, dass** man eine Verbindung der allgemeinen Formel (IV)

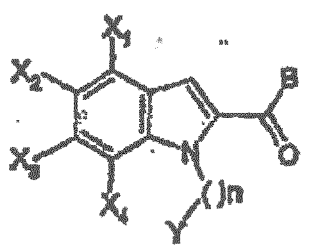

(IV)

worin $X_1$, $X_2$, $X_3$, $X_4$, Y und n die in der allgemeinen Formel (I) gemäß Anspruch 1 angegebene Bedeutung besitzen und für eine $C_1$-$C_6$-Alkoxygruppe steht, am Rückflusspunkt eines Lösungsmittels mit einem Amid der Verbindung der allgemeinen Formel (V)

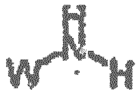

(V)

worin W die in der allgemeinen Formel (I) gemäß Anspruch 1 angegebene Bedeutung besitzt, umsetzt, wobei das Amid der Verbindung der allgemeinen Formel (V) durch vorherige Einwirkung von Trimethylaluminium auf die Amine der allgemeinen Formel (V) hergestellt wird.

**7.** Verbindung der Formel (IV), ausgewählt aus den folgenden Verbindungen:

- 5-Fluor-1-[(thiazol-2-yl)methyl]-1*H*-indol-2-carbonsäureethylester
- 5-Fluor-1-[(pyridin-3-yl)ethyl]-1*H*-indol-2-carbonsäureethylester
- 5-Fluor-1-[(pyridin-3-yl)methyl]-1*H*-indol-2-carbonsäureethylester
- 5-Fluor-1-[(pyridin-4-yl)methyl]-1*H*-indol-2-carbonsäureethylester
- 5-Fluor-1-[(pyridin-2-yl)methyl]-1*H*-indol-2-carbonsäureethylester
- 5-Fluor-1-[(2-methylpyridin-3-yl)methyl]-1*H*-indol-2-carbonsäureethylester
- 5-Fluor-1-[(1-N-benzylimidazol-2-yl)methyl]-1*H*-indol-2-carbonsäureethylester
- 5-Fluor-1-[(2-pyrrolidinopyridin-3-yl)methyl]-1*H*-indol-2-carbonsäureethylester
- 5-Fluor-1-[(benzothiazol-2-yl)methyl]-1*H*-indol-2-carbonsäureethylester
- 5-Fluor-1-[(1-methylbenzimidazol-2-yl)methyl]-1*H*-indol-2-carbonsäureethylester
- 5-Fluor-1-[(4-methylthiazol-5-yl)ethyl]-1*H*-indol-2-carbonsäureethylester
- 5-Fluor-1-[(chinolin-2-yl)methyl]-1*H*-indol-2-carbonsäureethylester
- 5-Fluor-1-[(chinoxalin-2-yl)methyl]-1*H*-indol-2-carbonsäureethylester
- 5-Fluor-1-[(pyrazin-2-yl)methyl]-1*H*-indol-2-carbonsäureethylester
- 5-Fluor-1-[(3-methylpyridin-2-yl)methyl]-1*H*-indol-2-carbonsäureethylester
- 5-Fluor-1-[(isochinolin-1-yl)methyl]-1*H*-indol-2-carbonsäureethylester
- 5-Fluor-1-[(4-methylpyridin-2-yl)methyl]-1*H*-indol-2-carbonsäureethylester
- 5-Fluor-1-[(2-methylpyridin-4-yl)ethyl]-1*H*-indol-2-carbonsäureethylester
- 5-Fluor-1-[(2-methylpyridin-4-yl)methyl]-1H-indol-2-carbonsäureethylester
- 5-Fluor-1-[(5-methylpyridin-2-yl)methyl]-1*H*-indol-2-carbonsäureethylester
- 5-Fluor-1-[(dimethylpyrazin-2-yl)methyl]-1*H*-indol-2-carbonsäureethylester
- 5-Fluor-1-[(6-methylpyrazin-2-yl)methyl]-1*H*-indol-2-carbonsäureethylester
- 5-Fluor-1-[(6-methylpyridazin-3-yl)methyl]-1*H*-indol-2-carbonsäureethylester
- 5-Fluor-1-[(6-methylpyridin-2-yl)methyl]-1*H*-indol-2-carbonsäureethylester
- 5-Fluor-1-[(2-phenylpyridin-4-yl)methyl]-1*H*-indol-2-carbonsäureethylester
- 6-tert.-Butyl-1-[(pyridin-4-yl)methyl]-1*H*-indol-2-carbonsäuremethylester
- 5-Trifluormethyl-1-[(pyridin-4-yl)methyl]-1*H*-indol-2-carbonsäureethylester
- 6-N-Dimethylamino-1-[(pyridin-4-yl)methyl]-1*H*-indol-2-carbonsäureethylester
- 6-Trifluormethyl-1-[(pyridin-4-yl)methyl]-1*H*-indol-2-carbonsäuremethylester
- 5-tert.-Butyl-1-[(pyridin-4-yl)methyl]-1*H*-indol-2-carbonsäureethylester
- 5-tert.-Butyl-1-[(pyridin-2-yl)methyl]-1*H*-indol-2-carbonsäureethylester
- 5-tert.-Butyl-1-[(pyridin-3-yl)methyl]-1*H*-indol-2-carbonsäureethylester
- 5-tert.-Butyl-1-[(2-methylpyridin-3-yl)methyl]-1*H*-indol-2-carbonsäureethylester
- 5-tert.-Butyl-1-[(6-methylpyridin-2-yl)methyl]-1*H*-indol-2-carbonsäureethylester
- 5-Fluor-1-[(pyrimidin-4-yl)methyl]-1*H*-indol-2-carbonsäureethylester

in Basen- oder Säureadditionssalzform sowie in Hydrat- oder Solvatform.

**8.** Verbindung der Formel (V), ausgewählt aus den folgenden Verbindungen:

- 5-Amino-1-(cyclopropyl)methyl-2-methyl-1*H*-benzimidazol
- 5-Amino-2-cyclopropyl-1-methyl-1*H*-benzimidazol
- 5-Amino-2-isopropyl-1-methyl-1*H*-benzimidazol
- 6-Amino-1-(cyclopropyl)methyl-2-methyl-1*H*-benzimidazol
- 6-Amino-2-cyclopropyl-1-methyl-1*H*-benzimidazol

- 6-Amino-2-isopropyl-1-methyl-1*H*-benzimidazol in Basen- oder Säureadditionssalzform sowie in Hydrat- oder Solvatform.

9. Arzneimittel, **dadurch gekennzeichnet, dass** es eine Verbindung der Formel (I) nach einem der Ansprüche 1 bis 5 oder ein pharmazeutisch unbedenkliches Salz oder auch ein Hydrat oder ein Solvat der Verbindung der Formel (I) umfasst.

10. Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz, ein Hydrat oder ein Solvat dieser Verbindung sowie mindestens einen pharmazeutisch unbedenklichen Hilfsstoff umfasst.

11. Verwendung einer Verbindung der Formel (I) nach einem der Ansprüche 1 bis 5 zur Herstellung eines Arzneimittels zur Prävention oder Behandlung von Pathologien, an denen die Rezeptoren vom TRPV1-Typ beteiligt sind.

12. Verwendung einer Verbindung der Formel (I) nach Anspruch 11 zur Herstellung eines Arzneimittels zur Prävention oder Behandlung von Schmerzen, Entzündungen, urologischen Störungen, gynäkologischen Störungen, gastrointestinalen Störungen, respiratorischen Störungen, Psoriasis, Pruritis, Haut-, Augen- oder Schleimhautreizungen, Herpes oder Zona oder zur Behandlung von Depression oder Diabetes.

## Claims

1. Compound corresponding to formula (I)

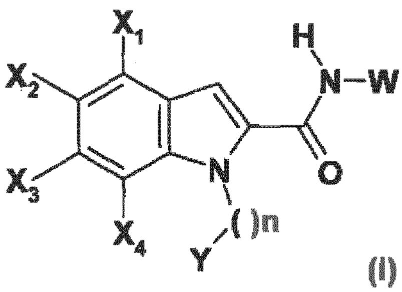

(I)

in which $X_1$, $X_2$, $X_3$, $X_4$ represent, independently of each other, a hydrogen or halogen atom or a $C_1$-$C_6$-alkyl, $C_1$-$C_6$-fluoroalkyl or $NR_1R_2$ group,

W represents a fused bicyclic group of formula:

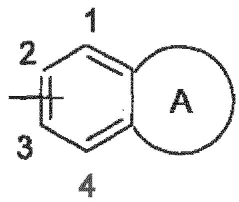

bonded to the nitrogen atom via positions 1, 2, 3 or 4;
A represents a 5- to 7-membered heterocycle comprising from one to three heteroatoms chosen from O, S and N;
the carbon atom(s) of A being optionally substituted with one or more groups chosen from a hydrogen atom and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl, aryl-$C_1$-$C_6$-alkylene, oxo or thio group;
the nitrogen atom(s) of A being optionally substituted with $R_6$ when the nitrogen is adjacent to a carbon atom substituted with an oxo group, or with $R_7$ in the other cases;
n is equal to 1, 2 or 3;
Y represents a heteroaryl chosen from pyridyl, pyrimidinyl, pyrazinyl, pyridazinyl, imidazolyl, benzimidazolyl, benzothiazolyl, thiazolyl, furyl, quinolyl, isoquinolyl, quinoxalinyl groups, the heteroaryl being optionally substituted with one or more groups chosen from $C_1$-$C_6$-alkyl, $C_1$-$C_6$-fluoroalkyl, aryl-$C_1$-$C_6$-alkylene and $NR_1R_2$ groups; $R_1$ and $R_2$ together forming, with the nitrogen atom that bears them, a morpholinyl group;
$R_1$ and $R_2$ represent, independently of each other, a hydrogen atom or a $C_1$-$C_6$-alkyl group;
$R_6$ represents a hydrogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-$C_1$-$C_6$-alkylene or aryl group;
$R_7$ represents a hydrogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-$C_1$-$C_6$-alkylene, $C_1$-$C_6$-alkyl-C(O)-, $C_3$-$C_7$-cycloalkyl-$C_1$-$C_3$-alkylene- (CO) -, $C_1$-$C_6$-fluoroalkyl-C (O)-, $C_3$-$C_7$-cycloalkyl-C(O)-, aryl-C(O)-, aryl-$C_1$-$C_6$-alkylene-C(O)-, $C_1$-$C_6$-alkyl-$S(O)_2$-, $C_1$-$C_6$-fluoroalkyl-S $(O)_2$-, $C_3$-$C_7$-cycloalkyl-$S(O)_2$-, $C_3$-$C_7$-cycloalkyl-$C_1$-$C_3$-alkylene-$S(O)_2$-, aryl-$S(O)_2$- or aryl-$C_1$-$C_6$-alkylene-S $(O)_2$- or aryl group;

the sulfur atom(s) of the heterocycle A or of the heteroaryl Y possibly being in oxidized form;
the nitrogen atom(s) of the heterocycle A or of the heteroaryl Y possibly being in oxidized form;
in the form of base or of acid-addition salt, and also in the form of hydrate or solvate.

**2.** Compound of formula (I) according to Claim 1, **characterized in that**
W represents a fused bicyclic group of formula:

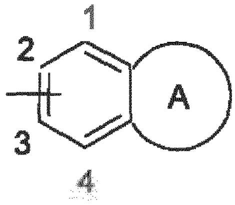

bonded to the nitrogen atom via positions 1, 2, 3 or 4; and W is chosen from indolinyl, isoindolyl, isoindolinyl, benzofuryl, dihydrobenzofuryl, benzothiophenyl, dihydrobenzothiophenyl, benzoxazolyl, dihydrobenzoxazolinyl, isobenzofuryl, dihydroisobenzofuryl, benzimidazolyl, dihydrobenzimidazolyl, indolyl, indazolyl, benzothiazolyl, isobenzothiazolyl, dihydroisobenzothiazolyl, benzotriazolyl, quinolyl, dihydroquinolyl, tetrahydroquinolyl, isoquinolyl, dihydroisoquinolyl, tetrahydroisoquinolyl, benzoxazinyl, dihydrobenzoxazinyl, benzothiazinyl, dihydrobenzothiazinyl, cinnolinyl, quinazolinyl, dihydroquinazolinyl, tetrahydroquinazolinyl, quinoxalinyl, dihydroquinoxalinyl, tetrahydroquinoxalinyl, phthalazinyl, dihydrophthalazinyl, tetrahydrophthalazinyl, tetrahydrobenz[*b*]azepinyl, tetrahydrobenz[*c*]azepinyl, tetrahydrobenz[*d*]azepinyl, tetrahydrobenzo[*b*][1,4]diazepinyl, tetrahydrobenzo[*e*][1,4]diazepinyl, tetrahydrobenzo[*b*][1,4]oxazepinyl or tetrahydrobenzo[*b*][1,4]thiazepinyl groups;
the carbon and/or nitrogen atom(s) of A being optionally substituted as defined in the general formula (I) according to Claim 1;
in the form of base or of acid-addition salt, and also in the form of hydrate or solvate.

**3.** Compound of formula (I) according to either of Claims 1 and 2, **characterized in that**
W represents a fused bicyclic group of formula:

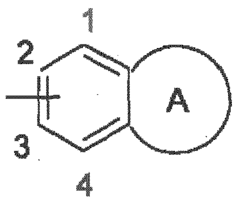

bonded to the nitrogen atom via positions 2 or 3;
and W is chosen from benzimidazolyl, indolyl, benzothiazolyl, quinolyl, tetrahydroquinolyl and benzoxazinyl groups; and/or
the carbon atom(s) of A being optionally substituted with one or more $C_1$-$C_6$-alkyl, $C_1$-$C_6$-fluoroalkyl, $C_3$-$C_7$-cycloalkyl or oxo groups; and/or
the nitrogen atom(s) of A being optionally substituted with $R_6$ when the nitrogen is adjacent to a carbon atom substituted with an oxo group, $R_6$ representing a hydrogen atom or a $C_1$-$C_6$-alkyl group; or with $R_7$ in the other cases, $R_7$ representing a $C_1$-$C_6$-alkyl group or a $C_3$-$C_7$-cycloalkyl-$C_1$-$C_3$-alkylene group;

in the form of base or of acid-addition salt, and also in the form of hydrate or solvate.

**4.** Compound of formula (I) according to any one of Claims 1 to 3, **characterized in that** n is equal to 1 or 2; in the form of base or of acid-addition salt, and also in the form of hydrate or solvate.

**5.** Compound of formula (I) according to Claim 1, **characterized in that**

$X_1$, $X_2$, $X_3$, $X_4$ represent, independently of each other, a hydrogen or halogen atom or a $C_1$-$C_6$-alkyl, $C_1$-$C_6$-fluoroalkyl or $NR_1R_2$ group, $R_1$ and $R_2$ being as defined in the general formula (I) according to Claim 1;
W represents a fused bicyclic group of formula:

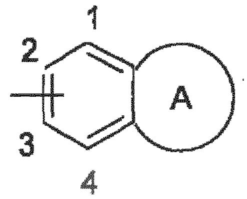

bonded to the nitrogen atom via positions 2 or 3; and
W is chosen from benzimidazolyl, indolyl, benzothiazolyl, quinolyl, tetrahydroquinolyl or benzoxazinyl groups; the carbon atom(s) of A being optionally substituted with one or more $C_1$-$C_6$-alkyl, $C_1$-$C_6$-fluoroalkyl, $C_3$-$C_7$-cycloalkyl or oxo groups; and/or
the nitrogen atom(s) of A being optionally substituted with $R_6$ when the nitrogen is adjacent to a carbon atom substituted with an oxo group, $R_6$ representing a hydrogen atom or a $C_1$-$C_6$-alkyl group; or with $R_7$ in the other cases, $R_7$ representing a $C_1$-$C_6$-alkyl group or a $C_3$-$C_7$-cycloalkyl-$C_1$-$C_3$-alkylene group; and/or
n is equal to 1 or 2; and/or
Y represents a heteroaryl chosen from pyridyl, pyrimidinyl, pyrazinyl, pyridazinyl, imidazolyl, benzimidazolyl, benzothiazolyl, thiazolyl, furyl, quinolyl, isoquinolyl, quinoxalinyl groups, the heteroaryl being optionally substituted with one or more groups chosen from $C_1$-$C_6$-alkyl, $C_1$-$C_6$-fluoroalkyl, aryl-$C_1$-$C_6$-alkylene and $NR_1R_2$ groups; $R_1$ and $R_2$ together forming, with the nitrogen atom that bears them, a morpholinyl group;

in the form of base or of acid-addition salt, and also in the form of hydrate or solvate.

**6.** Process for preparing a compound of general formula (I) according to Claim 1, **characterized in that** a compound of general formula (IV)

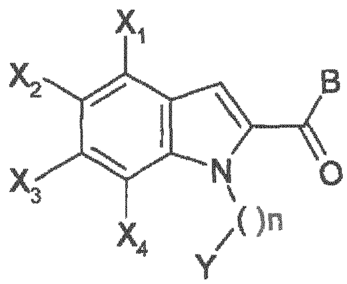

(IV)

in which $X_1$, $X_2$, $X_3$, $X_4$, Y and n are as defined in the general formula (I) according to Claim 1 and B represents a $C_1$-$C_6$-alkoxy group,
is reacted with an amide of the compound of general formula (V)

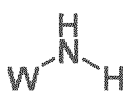

(V)

in which W is as defined in the general formula (I) according to Claim 1,
in a refluxing solvent, the amide of the compound of general formula (V) being prepared via the prior action of trimethylaluminium on the amines of general formula (V).

**7.** Compound of formula (IV) chosen from the following compounds:

- ethyl 5-fluoro-1-[(thiazol-2-yl)methyl]-1*H*-indole-2-carboxylate
- ethyl 5-fluoro-1-[(pyrid-3-yl)ethyl]-1*H*-indole-2-carboxylate
- ethyl 5-fluoro-1-[(pyrid-3-yl)methyl]-1*H*-indole-2-carboxylate
- ethyl 5-fluoro-1-[(pyrid-4-yl)methyl]-1*H*-indole-2-carboxylate
- ethyl 5-fluoro-1-[(pyrid-2-yl)methyl]-1*H*-indole-2-carboxylate
- ethyl 5-fluoro-1-[(2-methylpyrid-3-yl)methyl]-1*H*-indole-2-carboxylate
- ethyl 5-fluoro-1-[(1-N-benzylimidazol-2-yl)methyl]-1*H*-indole-2-carboxylate
- ethyl 5-fluoro-1-[(2-pyrrolidinopyrid-3-yl)methyl]-1*H*-indole-2-carboxylate
- ethyl 5-fluoro-1-[(benzothiazol-2-yl)methyl]-1*H*-indole-2-carboxylate
- ethyl 5-fluoro-1-[(1-methylbenzimidazol-2-yl)methyl]-1*H*-indole-2-carboxylate
- ethyl 5-fluoro-1-[(4-methylthiazol-5-yl)ethyl]-1*H*-indole-2-carboxylate
- ethyl 5-fluoro-1-[(quinol-2-yl)ethyl]-1*H*-indole-2-carboxylate
- ethyl 5-fluoro-1-[(quinoxalin-2-yl)methyl]-1*H*-indole-2-carboxylate
- ethyl 5-fluoro-1-[(pyrazin-2-yl)methyl]-1*H*-indole-2-carboxylate
- ethyl 5-fluoro-1-[(3-methylpyrid-2-yl)methyl]-1*H*-indole-2-carboxylate
- ethyl 5-fluoro-1-[(isoquinol-1-yl)methyl]-1*H*-indole-2-carboxylate
- ethyl 5-fluoro-1-[(4-methylpyrid-2-yl)methyl]-1*H*-indole-2-carboxylate
- ethyl 5-fluoro-1-[(2-methylpyrid-4-yl)ethyl]-1*H*-indole-2-carboxylate
- ethyl 5-fluoro-1-[(2-methylpyrid-4-yl)methyl]-1*H*-indole-2-carboxylate
- ethyl 5-fluoro-1-[(5-methylpyrid-2-yl)methyl]-1*H*-indole-2-carboxylate
- ethyl 5-fluoro-1-[(dimethylpyrazin-2-yl)methyl]-1*H*-indole-2-carboxylate
- ethyl 5-fluoro-1-[(6-methylpyrazin-2-yl)methyl]-1*H*-indole-2-carboxylate
- ethyl 5-fluoro-1-[(6-methylpyridazin-3-yl)methyl]-1*H*-indole-2-carboxylate
- ethyl 5-fluoro-1-[(6-methylpyrid-2-yl)methyl]-1*H*-indole-2-carboxylate
- ethyl 5-fluoro-1-[(2-phenylpyrid-4-yl)methyl]-1*H*-indole-2-carboxylate
- methyl 6-tert-butyl-1-[(pyrid-4-yl)methyl]-1*H*-indole-2-carboxylate
- ethyl 5-trifluoromethyl-1-[(pyrid-4-yl)methyl]-1*H*-indole-2-carboxylate
- ethyl 6-N-dimethylamino-1-[(pyrid-4-yl)methyl]-1*H*-indole-2-carboxylate
- methyl 6-trifluoromethyl -1-[(pyrid-4-yl)methyl]-1*H*-indole-2-carboxylate
- ethyl 5-tert-butyl-1-[(pyrid-4-yl)methyl]-1*H*-indole-2-carboxylate
- ethyl 5-terbutyl-1-[(pyrid-2-yl)methyl]-1*H*-indole-2-carboxylate
- ethyl 5-terbutyl-1-[(pyrid-3-yl)methyl]-1*H*-indole-2-carboxylate
- ethyl 5-terbutyl-1-[(2-methylpyrid-3-yl)methyl]-1*H*-indole-2-carboxylate
- ethyl 5-terbutyl-1-[(6-methylpyrid-2-yl)methyl]-1*H*-indole-2-carboxylate
- ethyl 5-fluoro-1-[(pyrimidin-4-yl)methyl]-1*H*-indole-2-carboxylate

in the form of base or of acid-addition salt, and also in the form of hydrate or solvate.

**8.** Compound of formula (V) chosen from the following compounds:

- 5-amino-1-(cyclopropyl)methyl-2-methyl-1*H*-benzimidazole
- 5-amino-2-cyclopropyl-1-methyl-1*H*-benzimidazole
- 5-amino-2-isopropyl-1-methyl-1*H*-benzimidazole
- 6-amino-1-(cyclopropyl)methyl-2-methyl-1*H*-benzimidazole
- 6-amino-2-cyclopropyl-1-methyl-1*H*-benzimidazole
- 6-amino-2-isopropyl-1-methyl-1*H*-benzimidazole in the form of base or of acid-addition salt, and also in the form of hydrate or solvate.

**9.** Medicament, **characterized in that** it comprises a compound of formula (I) according to any one of Claims 1 to 5, or a pharmaceutically acceptable salt, or alternatively a hydrate or solvate of the compound of formula (I),

**10.** Pharmaceutical composition, **characterized in that** it comprises a compound of formula (I) according to any one

of Claims 1 to 5, or a pharmaceutically acceptable salt, a hydrate or a solvate of this compound, and also at least one pharmaceutically acceptable excipient.

**11.** Use of a compound of formula (I) according to any one of Claims 1 to 5 for the preparation of a medicament for preventing or treating pathologies in which the TRPV1 type receptors are involved.

**12.** Use of a compound of formula (I). according to Claim 11 for the preparation of a medicament for preventing or treating pain, inflammation, urological disorders, gynaecological disorders, gastrointestinal disorders, respiratory disorders, psoriasis, pruritus, dermal, ocular or mucous irritation, herpes or zona, or for treating depression or diabetes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03068749 A
- WO 2004072069 A
- JP 2001151771 A
- WO 03049702 A
- WO 2002059110 A
- WO 02059110 A
- WO 9604270 A


### Littérature non-brevet citée dans la description


- **J. MARCH.** Advances in Organic Chemistry. Wiley Interscience, 2001
- **D. KNITTEL.** *Synthesis,* 1985, vol. 2, 186
- **T.M. WILLIAMS.** *J.Med.Chem.,* 1993, vol. 36 (9), 1291
- **KOLASA T.** *Bioorg.Med.Chem.,* 1997, vol. 5 (3), 507
- **ABRAMOVITCH R.** *Synth. Commun.,* 1995, vol. 25 (1), 1
- **O. MITSONOBU.** *Synthesis,* 1981, 1-28
- **CARLING R.W. et al.** *J.Med.Chem.,* 2004, vol. 47, 1807-1822
- **RUSSEL M.G.N. et al.** *J.Med.Chem.,* 2005, vol. 48, 1367-1383
- **I.T. FORBES.** *J.Med.Chem.,* 1993, vol. 36 (8), 1104